# EUROPEAN PATENT APPLICATION

(11) **EP 4 835 087 A1**
(43) Date of publication of application: **04.11.2026**
(21) Application number: 25736186.5
(22) Date of filing: 02.01.2025
(51) Int. Cl.: C12N 15/85, C12N 15/13, A01K 67/0278, C12N 5/10, C07K 16/28, C07K 16/00

(54) **ANIMAL MODEL AND CONSTRUCTION METHOD THEREFOR**

(30) Priority: 02.01.2024 WO PCT/CN2024/070020
(71) Applicant: Gempharmatech Co., Ltd, Nanjing, Jiangsu 210061 (CN)
(72) Inventor: ZHANG, Mingkun, Nanjing, Jiangsu 210061 (CN); LI, Song, Nanjing, Jiangsu 210061 (CN); ZHANG, Yuxi, Nanjing, Jiangsu 210061 (CN); PENG, Junchao, Nanjing, Jiangsu 210061 (CN); LIANG, Juan, Nanjing, Jiangsu 210061 (CN); ZHAO, Jing, Nanjing, Jiangsu 210061 (CN); GAO, Xiang, Nanjing, Jiangsu 210061 (CN)
(74) Representative: Gong, Jinping
(86) International application number: PCT/CN2025/070158
(87) International publication number: WO 2025/146085

(57) **Abstract**

The present application relates to a non-human animal and a method for preparing the non-human animal. In the genome of the non-human animal according to the present application, the CH1 position in the immunoglobulin heavy chain constant region gene is changed. More specifically, the CH1 position is changed such that the exon order is: CH2, CH3 and CH1 in sequence.

## Description

### TECHNICAL FIELD

The present application relates to the technical field of biomedicine, and specifically to a non-human animal model for producing an antibody and a construction method thereof, particularly a genetically modified non-human animal that retains an immunoglobulin CH1 region to produce a heavy chain antibody.

### BACKGROUND

Monoclonal antibodies have completely changed the treatment of cancers. However, the large size (150 kDa) of traditional antibodies leads to poor penetration into solid tumors and undesirable therapeutic effects. Nano-antibodies (15 kDa) and human heavy-chain only antibodies (75 kDa) based on nano-antibodies have the advantages of small volume, high solubility, high stability and good tissue permeability in vivo.

The discovery of heavy chain antibodies has brought unprecedented opportunities for cancer treatment. A conventional antibody consists of paired heavy and light chains. The absence of heavy or light chain expression will lead to arrested B cell development. Some species, such as camels (dromedaries/llamas) and sharks, can produce heavy chain-only antibodies (HcAbs). These unique forms of camel-derived antibodies lack complete light chain and CH1 domain.

HcAbs are screened by utilizing the characteristics of camel that produce heavy-chain only antibodies, but the heavy chain-only antibodies screened by this method require further humanization due to the immunogenicity. The humanization process may adversely affect the binding affinity and introduce an immunogenic epitope to the antibodies. Iterative and time-consuming experiments are usually needed to improve the characteristics of these antibodies. In some cases, these antibodies may also cause immunogenicity in patients, resulting in the weakening of their efficacy over time. A good solution is to screen heavy-chain only antibodies using a transgenic humanized HcAbs rodent model, with which the laborious humanization step required for the camel-derived heavy-chain only antibodies can be avoided by in-vivo screening. In addition, compared with the Camelidae HcAb discovery platform, the transgenic rodent platform for HcAbs also has some other advantages, such as shorter immunization time, easier access and operation compared with large Camelidae, more readily availability of immune organ materials in the body, more consistent immune response, and more sophisticated hybridoma fusion or display and other antibody discovery technologies. At present, there are mainly three strategies for the preparation of transgenic rodent models for human HcAbs (mainly mice). The first strategy is to produce heavy-chain only antibodies by silencing or knocking out the mouse light chain so that it is defective and cannot bind to the heavy chain. However, in these mice, the development of B cells is arrested in the immature B cell phase, which affects the screening of downstream antibodies. The second strategy is to construct an expression vector of human VDJ+ mouse CH1 deleted constant region for transgenesis. This random insertion method has obvious disadvantages. The expression of the target gene is easily affected by a flanking sequence at the random insertion position, and there is the risk of destroying other endogenous genes. Moreover, this strategy generally needs to silence the whole endogenous heavy chain, leading to abnormal development of B cells. The third strategy is to produce heavy-chain only antibodies by the deletion of endogenous CH1 sequence, but the existence of CH1 exon is quite important. For example, a) CH1 has an allotypic genetic marker thereon (Ternant, David et al. "IgG1 Allotypes Influence the Pharmacokinetics of Therapeutic Monoclonal Antibodies through FcRn Binding." Journal of immunology (Baltimore, Md.: 1950) vol. 196,2 (2016): 607-13); and b) the presence of CH1 enables quality-control screening of proteins that are not folded correctly (Matthias J, Feige, Sandra, Groscurth, Moritz, Marcinowski et al. An unfolded CH1 domain controls the assembly and secretion of IgG antibodies.[J] .Mol Cell, 2009, 34: 0). Therefore, the deletion of CH1 exon has potential adverse effects. There is a need in the industry for a non-human animal model that retains the CH1 exon and produces heavy chain antibodies, to effectively and efficiently produce humanized or non-humanized antibodies.

### SUMMARY

The present application provides a non-human animal and a method for preparing the non-human animal. In the method, the CH1 position is changed by editing the immunoglobulin heavy chain constant region gene in the genome of the non-human animal. The non-human animal in the present application can avoid the interference with the gene expression and regulation in the animal per se, avoid the risk that the deletion of CH1 destroys other endogenous genes and causes the abnormal development of B cells, and have great advantages in antibody production. The non-human animal (for example, mouse) in the present application has a functionally humanized heavy-chain only antibody, and a good solution is created by screening a heavy-chain only antibody using the non-human animal. With the model for humanized heavy-chain only antibodies, the laborious humanization step required for the camel-derived heavy-chain only antibodies can be avoided by in-vivo screening. In addition, compared with the Camelidae HcAb discovery platform, the mouse model for humanized heavy-chain only antibodies further has other advantages, such as shorter immunization time, easier access and operation compared with large Camelidae, more ready availability of immune organ materials in the body, more consistent immune response, and more sophisticated hybridoma fusion or display and other antibody discovery technologies.

In one aspect, the present application provides a method for preparing a non-human animal. In the method, the CH1 position is changed by editing the immunoglobulin heavy chain constant region gene in the genome of the non-human animal.

In certain embodiments, the immunoglobulin heavy chain constant region gene includes the following exons: CH2, CH3 and CH1; and the exon order is CH2, CH3 and CH1 in sequence.

In certain embodiments, the immunoglobulin heavy chain constant region gene includes IgM, IgD, IgG, IgA or IgE. In certain embodiments, the immunoglobulin heavy chain constant region gene is IgG. In certain embodiments, the immunoglobulin heavy chain constant region gene is IgM. In certain embodiments, the immunoglobulin heavy chain constant region gene is IgD. In certain embodiments, the immunoglobulin heavy chain constant region gene is IgA. In certain embodiments, the immunoglobulin heavy chain constant region gene is IgA.

In certain embodiments, the IgG includes IgG3, IgG1, IgG2A, IgG2B and/or IgG2C.

In certain embodiments, the immunoglobulin heavy chain constant region gene further includes the following exons: H, M1 and M2, and a gene L encoding a linker; and the exon order is H, CH2, CH3, M1, M2, L and CH1 in sequence.

In certain embodiments, the linker is a self-cleaving peptide, and preferably a self-cleaving peptide having 18-22 amino acids in length.

In certain embodiments, the linker is a 2A peptide, and preferably F2A, E2A, P2A and T2A.

In certain embodiments, the immunoglobulin heavy chain constant region gene is derived from the non-human animal.

In certain embodiments, the method includes constructing a nucleic acid sequence, where the nucleic acid sequence includes sequentially, from the 5' to 3' end, the following exons: CH2, CH3 and CH1. In certain embodiments, the nucleic acid sequence includes sequentially, from the 5' to 3' end, the following exons and a gene L encoding a linker: H, CH2, CH3, M1, M2, L, and CH1.

In certain embodiments, the method includes inserting the nucleic acid sequence into the immunoglobulin heavy chain constant region gene locus of the non-human animal.

In certain embodiments, the method includes inserting the nucleic acid sequence into the immunoglobulin heavy chain constant region IGHG gene locus of the non-human animal.

In certain embodiments, the method includes replacing the endogenous immunoglobulin heavy chain constant region IGHG gene of the non-human animal by the nucleic acid sequence.

In certain embodiments, the method includes operably linking a human immunoglobulin heavy chain variable region gene downstream of the immunoglobulin heavy chain gene locus of the non-human animal.

In certain embodiments, the method includes operably linking the gene(s) of one or multiple human heavy chain variable region V region(s), heavy chain variable region D region(s) or heavy chain variable region J region(s), or fragment(s) thereof downstream of the heavy chain constant region gene locus of the non-human animal. In certain embodiments, the genes of the multiple human heavy chain variable region V regions, heavy chain variable region D regions, or heavy chain variable region J regions, or fragments thereof are directly linked.

In certain embodiments, the non-human animal includes an endogenous immunoglobulin heavy chain variable region gene. In certain embodiments, the genomic integrity of the endogenous immunoglobulin heavy chain variable region gene sequence of the non-human animal remains unchanged. In certain embodiments, the expression regulatory element for the endogenous immunoglobulin heavy chain variable region gene of the non-human animal is functionally unimpaired. In certain embodiments, the non-human animal includes an expression regulatory element for complete endogenous immunoglobulin heavy chain variable region gene. In certain embodiments, the non-human animal includes complete endogenous immunoglobulin heavy chain variable region gene.

In certain embodiments, the non-human animal does not express the functional endogenous immunoglobulin heavy chain variable region.

In certain embodiments, the endogenous immunoglobulin variable region gene of the non-human animal does not express the antibody heavy chain variable region.

In certain embodiments, the transcription direction of the human immunoglobulin heavy chain variable region gene is reverse to that of the endogenous immunoglobulin heavy chain variable region gene.

In certain embodiments, the immunoglobulin heavy chain constant region gene of the non-human animal is included between the human immunoglobulin heavy chain variable region gene and the endogenous immunoglobulin heavy chain variable region gene in the non-human animal.

In certain embodiments, the transcription direction of the immunoglobulin heavy chain constant region gene is reverse to that of the endogenous immunoglobulin heavy chain variable region gene.

In certain embodiments, the transcription direction of the human immunoglobulin heavy chain variable region gene is the same as that of the immunoglobulin heavy chain constant region gene in the non-human animal.

In certain embodiments, the chromosome of the non-human animal includes sequentially, from upstream to downstream, the endogenous immunoglobulin heavy chain variable region gene, the immunoglobulin heavy chain constant region gene, and the human immunoglobulin heavy chain variable region gene.

In certain embodiments, the non-human animal is a rodent. In certain embodiments, the non-human animal is a mouse.

In certain embodiments, the human immunoglobulin heavy chain variable region gene is inserted between the chromosome locations chr12: 113,149,523-113,223,857 of the mouse. In certain embodiments, the human immunoglobulin heavy chain variable region gene is inserted at the chromosome location chr12: 113,190,256 of the mouse.

In certain embodiments, the method includes operably linking the human immunoglobulin heavy chain variable region gene downstream of the immunoglobulin heavy chain gene locus of the non-human animal by site-specific recombination. In certain embodiments, the method enables the transcription direction of the immunoglobulin heavy chain constant region gene of the non-human animal to be reverse to that of the endogenous immunoglobulin heavy chain variable region gene through genome modification.

In certain embodiments, the method includes operably linking a human immunoglobulin light chain variable region gene downstream of the immunoglobulin light chain gene locus of the non-human animal.

In certain embodiments, the method includes operably linking the gene(s) of one or multiple human light chain variable region V region(s) or light chain variable region J region(s), or fragment(s) thereof downstream of the light chain constant region gene locus of the non-human animal. In certain embodiments, the gene(s) of the one or multiple human light chain variable region V region(s) or light chain variable region J region(s), or fragment(s) is/are directly linked.

In certain embodiments, the non-human animal includes an endogenous immunoglobulin light chain variable region gene. In certain embodiments, the genomic integrity of the endogenous immunoglobulin light chain variable region gene sequence of the non-human animal remains unchanged. In certain embodiments, the expression regulatory element for the endogenous immunoglobulin light chain variable region gene of the non-human animal is functionally unimpaired. In certain embodiments, the non-human animal includes an expression regulatory element for complete endogenous immunoglobulin light chain variable region gene. In certain embodiments, the non-human animal includes complete endogenous immunoglobulin light chain variable region gene.

In certain embodiments, the non-human animal does not express the functional endogenous immunoglobulin light chain variable region.

In certain embodiments, the endogenous immunoglobulin variable region gene of the non-human animal does not express the antibody light chain variable region.

In certain embodiments, the transcription direction of the human immunoglobulin light chain variable region gene is reverse to that of the endogenous immunoglobulin light chain variable region gene.

In certain embodiments, the immunoglobulin light chain constant region gene of the non-human animal is included between the human immunoglobulin light chain variable region gene and the endogenous immunoglobulin light chain variable region gene in the non-human animal.

In certain embodiments, the transcription direction of the immunoglobulin light chain constant region gene is reverse to that of the endogenous immunoglobulin light chain variable region gene.

In certain embodiments, the transcription direction of the human immunoglobulin light chain variable region gene is the same as that of the immunoglobulin light chain constant region gene in the non-human animal.

In certain embodiments, the distance between the human immunoglobulin light chain variable region gene and the endogenous immunoglobulin light chain variable region gene is 4 Kbp-42 Kbp.

In certain embodiments, the non-human animal is a rodent. In certain embodiments, the non-human animal is a mouse.

In certain embodiments, the human immunoglobulin light chain variable region gene is inserted between the chromosome locations chr6: 70,703,738-70,742,704 of the mouse. In certain embodiments, the human immunoglobulin light chain variable region gene is inserted at the chromosome location chr6: 70,706,267 of the mouse.

In certain embodiments, the method includes operably linking the human immunoglobulin light chain variable region gene downstream of the immunoglobulin light chain gene locus of the non-human animal by site-specific recombination. In certain embodiments, the method enables the transcription direction of the immunoglobulin light chain constant region gene of the non-human animal to be reverse to that of the endogenous immunoglobulin light chain variable region gene through genome modification.

In another aspect, the present application provides a non-human animal or non-human animal cell.

In another aspect, the present application further provides an offspring of the non-human animal. The offspring can be an offspring of the non-human animal mating with the same genotype or other genotypes.

In another aspect, the present application further provides cells (for example, stem cells, embryonic stem cells, immune cells, B cells, T cells or hybridomas) derived from the non-human animal or an offspring thereof, or cell lines, or primary cell cultures thereof.

In another aspect, the present application provides a non-human animal, the immunoglobulin heavy chain constant region gene in the genome of the non-human animal includes the following exons: CH2, CH3 and CH1, and the exon order is CH2, CH3 and CH1 in sequence.

In another aspect, the present application provides a genome of a non-human animal. The immunoglobulin heavy chain constant region gene in the genome of the non-human animal includes the following exons: CH2, CH3 and CH1, and the exon order is CH2, CH3 and CH1 in sequence.

In certain embodiments, CH2, CH3 and CH1 are derived from IgG and/or IgM.

In certain embodiments, the IgG includes IgG3, IgG1, IgG2A, IgG2B and/or IgG2C.

In certain embodiments, the immunoglobulin heavy chain constant region gene further includes the following exons: H, M1 and M2, and a gene L encoding a linker; and the exon order is H, CH2, CH3, M1, M2, L and CH1 in sequence.

In certain embodiments, the linker is a self-cleaving peptide, and preferably a self-cleaving peptide having 18-22 amino acids in length.

In certain embodiments, the linker is a 2A peptide, and preferably F2A, E2A, P2A and T2A.

In certain embodiments, the immunoglobulin heavy chain constant region gene is derived from the non-human animal.

In certain embodiments, a human immunoglobulin heavy chain variable region gene is operably linked downstream of the immunoglobulin heavy chain gene locus of the non-human animal. In certain embodiments, the gene(s) of one or multiple human heavy chain variable region V region(s), heavy chain variable region D region(s) or heavy chain variable region J region(s), or fragment(s) thereof is/are operably linked downstream of the heavy chain constant region gene locus of the non-human animal. In certain embodiments, the genes of the multiple human heavy chain variable region V regions, heavy chain variable region D regions, or heavy chain variable region J regions, or fragments thereof are directly linked.

In certain embodiments, the non-human animal includes an endogenous immunoglobulin heavy chain variable region gene. In certain embodiments, the genomic integrity of the endogenous immunoglobulin heavy chain variable region gene sequence of the non-human animal remains unchanged. In certain embodiments, the expression regulatory element for the endogenous immunoglobulin heavy chain variable region gene of the non-human animal is functionally unimpaired. In certain embodiments, the non-human animal includes an expression regulatory element for complete endogenous immunoglobulin heavy chain variable region gene. In certain embodiments, the non-human animal includes complete endogenous immunoglobulin heavy chain variable region gene.

In certain embodiments, the non-human animal does not express the functional endogenous immunoglobulin heavy chain variable region. In certain embodiments, the endogenous immunoglobulin variable region gene of the non-human animal does not express the antibody heavy chain variable region.

In certain embodiments, the transcription direction of the human immunoglobulin heavy chain variable region gene is reverse to that of the endogenous immunoglobulin heavy chain variable region gene.

In certain embodiments, the immunoglobulin heavy chain constant region gene of the non-human animal is included between the human immunoglobulin heavy chain variable region gene and the endogenous immunoglobulin heavy chain variable region gene in the non-human animal.

In certain embodiments, the transcription direction of the immunoglobulin heavy chain constant region gene is reverse to that of the endogenous immunoglobulin heavy chain variable region gene.

In certain embodiments, the transcription direction of the human immunoglobulin heavy chain variable region gene is the same as that of the immunoglobulin heavy chain constant region gene in the non-human animal.

In certain embodiments, the chromosome of the non-human animal includes sequentially, from upstream to downstream, the endogenous immunoglobulin heavy chain variable region gene, the immunoglobulin heavy chain constant region gene, and the human immunoglobulin heavy chain variable region gene.

In certain embodiments, the non-human animal is a rodent. In certain embodiments, the non-human animal is a mouse.

In certain embodiments, the human immunoglobulin heavy chain variable region gene is inserted between the chromosome locations chr12: 113,149,523-113,223,857 of the mouse. In certain embodiments, the human immunoglobulin heavy chain variable region gene is inserted at the chromosome location chr12: 113,190,256 of the mouse.

In certain embodiments, a human immunoglobulin light chain variable region gene is operably linked downstream of the immunoglobulin light chain gene locus of the non-human animal.

In certain embodiments, the method includes operably linking the gene(s) of one or multiple human light chain variable region V region(s) or light chain variable region J region(s), or fragment(s) thereof downstream of the light chain constant region gene locus of the non-human animal. In certain embodiments, the gene(s) of the one or multiple human light chain variable region V region(s) or light chain variable region J region(s), or fragment(s) is/are directly linked.

In certain embodiments, the non-human animal includes an endogenous immunoglobulin light chain variable region gene. In certain embodiments, the genomic integrity of the endogenous immunoglobulin light chain variable region gene sequence of the non-human animal remains unchanged. In certain embodiments, the expression regulatory element for the endogenous immunoglobulin light chain variable region gene of the non-human animal is functionally unimpaired. In certain embodiments, the non-human animal includes an expression regulatory element for complete endogenous immunoglobulin light chain variable region gene. In certain embodiments, the non-human animal includes complete endogenous immunoglobulin light chain variable region gene.

In certain embodiments, the non-human animal does not express the functional endogenous immunoglobulin light chain variable region.

In certain embodiments, the endogenous immunoglobulin variable region gene of the non-human animal does not express the antibody light chain variable region.

In certain embodiments, the transcription direction of the human immunoglobulin light chain variable region gene is reverse to that of the endogenous immunoglobulin light chain variable region gene.

In certain embodiments, the chromosome of the non-human animal includes sequentially, from upstream to downstream, the endogenous immunoglobulin light chain variable region gene, the immunoglobulin light chain constant region gene, and the human immunoglobulin light chain variable region gene.

In certain embodiments, the transcription direction of the immunoglobulin light chain constant region gene is reverse to that of the endogenous immunoglobulin light chain variable region gene.

In certain embodiments, the transcription direction of the human immunoglobulin light chain variable region gene is the same as that of the immunoglobulin light chain constant region gene in the non-human animal.

In certain embodiments, the distance between the human immunoglobulin light chain variable region gene and the endogenous immunoglobulin light chain variable region gene is 4 Kbp-42 Kbp.

In certain embodiments, the non-human animal is a mouse, and the human immunoglobulin light chain variable region gene is inserted between the chromosome locations chr6: 70,703,738-70,742,704. In certain embodiments, the non-human animal is a mouse, and the human immunoglobulin light chain variable region gene is inserted at the chromosome location chr6: 70,706,267.

In another aspect, the present application provides an engineered chromosome, which includes the genome of the non-human animal according to the present application.

In another aspect, the present application provides a non-human animal cell, which has a genome with an immunoglobulin heavy chain constant region gene including the following exons: CH2, CH3, CH1, H, M1, and M2; and a gene L encoding a linker; and the exon order is H, CH2, CH3, M1, M2, L, and CH1 in sequence.

In another aspect, the present application provides a method for preparing an antibody that specifically binds to an antigen. The method includes: exposing the non-human animal or cell to the antigen, and producing a hybridoma with cells collected from the animal. The method further includes: collecting a chimeric antibody produced by the hybridoma. The method may further include: sequencing the variable region gene of the hybridoma.

In certain embodiments, the antibody is a heavy chain-only antibody (HcAb).

Moreover, the present application provides a method for preparing a sample. The method includes: exposing the non-human animal according to the present application to an antigen; and collecting the sample from the non-human animal. In certain embodiments, the sample includes immune cells, such as B cells. In certain embodiments, the sample includes bone marrow, spleen tissues, lymph nodes, spleen cells or peripheral lymphocytes.

The technical solutions of the present application include one or more of the following advantages:
The non-human animal according to the present application produces an antibody without a light chain, that is, a heavy chain antibody. Because the heavy chain antibody has no light chain, it is smaller and thus expected to show better tissue penetration than an antibody containing a light chain, it has a similar or more favorable pharmacokinetic profile and still retain similar effector functions compared with conventional antibodies. Because the heavy chain antibody is small, it can also be administered at a higher dose in a given volume. The common method for the administration of antibodies is subcutaneous injection. For a given dose of an antibody, reducing the volume of administration can provide benefits to the patients and avoid the complications and pain caused by the subcutaneous injection in a large volume. Another advantage of the heavy chain antibody is that a bispecific antibody can be produced by heterodimerization of heavy chains specific for two different epitopes in a single therapeutic agent. Because the heavy chain antibody has no light chain, it is especially suitable for use in the production of a bispecific antibody, since the ordinary light chain that does not interfere with the binding affinity or specificity of any heavy chain has no need to be engineered, and the bispecific antibody can still be expressed properly.

The non-human animal described in the present application has advantages in avoiding interfering with the gene expression and regulation in the animal per se. For example, the deletion of CH1 exon in the immunoglobulin heavy chain constant region gene in the non-human animal (e.g., mouse) is avoided. The existence of CH1 exon in the non-human animal is critical, for example, CH1 has an allotypic genetic marker thereon. IgG1 isotypes affect the pharmacokinetics of therapeutic monoclonal antibodies through FcRn binding. The type of the CH1 domain affects B-cell receptor (BCR) assembly and expression, and antigen sensing. By means of the presence of CH1, the protein that is not folded correctly can be screened out under quality control.

The non-human animal described in the present application has advantages in avoiding interfering with the gene expression and regulation in the animal per se. For example, the human variable region gene (e.g., light chain variable region, heavy chain variable region, or light chain variable region and heavy chain variable region) is inserted downstream of (after) the endogenous immunoglobulin gene locus in mouse cells to avoid the insertion of a large-fragment DNA sequence or the deletion of a DNA sequence in the endogenous immunoglobulin gene locus of the non-human animal (e.g., mice). There is a very long "Junk sequence" downstream of the endogenous immunoglobulin gene locus in the non-human animals (for example, mice). For example, according to the recently publicized GRCm39 mouse full-wide sequence reference, there are a reported sequence of a 66 kb-long nonfunctional gene downstream of (after) the mouse immunoglobulin heavy chain gene and a reported sequence of a 39 kb-long nonfunctional gene downstream of (after) the mouse immunoglobulin κ light chain gene. Introducing a large-fragment foreign gene into these long "Junk sequences" can avoid interfering with the expression and regulation of endogenous genes in the non-human animal (such as mice) from the genome level.

The non-human animal described in the present application further has advantages in avoiding interfering with the gene expression and regulation in the animal per se. For example, the modification or deletion of immunoglobulin variable region gene of the non-human animal (e.g., mice) is avoided. Because there are many functional protein coding genes and functional microRNA genes in the endogenous immunoglobulin variable region gene locus of the non-human animal (for example, mice), the deletion or modification of these genes may result in unpredictable phenotypes of the animal. For example, the mouse immunoglobulin heavy chain gene locus contains the functional protein coding gene Adam6a, and the modification or deletion of Adam6a gene will lead to sterility of male mice.

The non-human animal described in the present application has advantages in producing antibodies. In the non-human animal described in the present application, both the immunoglobulin variable region gene and constant region gene in the host non-human animal are preserved. That is, both the endogenous immunoglobulin variable region gene and constant region gene in the non-human animal are preserved. All the immunoglobulin regulatory sequences in the host, including a promoter, an enhancer, a transition region and other potential expression regulatory sequences, are preserved. Therefore, the recombination and expression of immunoglobulin gene, B cell development, affinity maturation and the like in the non-human animal can be well ensured.

The non-human animal described in the present application has advantages in producing antibodies. For example, in some embodiments, the constant region of endogenous immunoglobulin in an animal is totally or partly modified (for example, the constant region is reversed totally or partly), so as to have a transcription direction reverse to that of the endogenous variable region gene. This avoids the V(D)J recombination between the endogenous variable region and the endogenous constant region to produce an antibody.

The non-human animal described in the present application further has advantages in producing antibodies. For example, in some embodiments, the inserted foreign human immunoglobulin variable region is away from the endogenous variable region, and has a reverse transcription direction, to avoid the integration of the endogenous variable region into VDJ recombination or VJ recombination with the human variable region due to modification and recombination by a DNA recombinase, and further avoid the production of antibodies with immunogenic epitopes in human caused by an endogenous variable region pedigree to a great extent.

The non-human animal described in the present application further has advantages in producing antibodies. For example, in some embodiments, all fragments of the human variable region gene are introduced and inserted stepwise downstream of (after) the endogenous immunoglobulin gene locus in the cells of the non-human animal (for example, mice). The variable domain produced by the obtained non-human animal can have almost the same diversity as that of the variable domain in human. This also means that the obtained non-human animal has an antibody diversity as close as possible to the antibody diversity in the human body per se and the possibility of obtaining high-affinity specific antibodies.

The method for preparing the non-human animal described in the present application also has advantages. For example, in some embodiments, the endogenous immunoglobulin constant region of the non-human animal (for example, mice) is totally or partly modified, so as to have a transcription direction reverse to that of the endogenous variable region gene. Then the modified human variable region gene is inserted downstream of (after) the endogenous immunoglobulin gene locus in the cells of the non-human animal (for example, mice). The human variable region gene can be inserted fragment by fragment from back to front in a direction reverse to the transcription direction of the immunoglobulin gene locus in the animal. As a result, the modified cells with part of the human variable region obtained by inserting almost every fragment can be injected into the early embryo of the animal to prepare a chimeric animal (for example, mice), produce or breed the non-human animal. In the obtained non-human animal, VDJ or VJ recombination with the inserted part of human variable region gene occurs, and chimeric antibodies with functional human variable region are produced. The non-human animal carrying part of the human variable region can be used as a checkpoint to make a non-human animal with complete human variable region. The reliability and functionality of fragment-by-fragment insertion of the human variable region are determined from the antibody production level and the structural diversity of the variable region, and the success or failure prediction and route correction for preparing the non-human animal with complete human variable region are performed timely. The non-human animal carrying part of the human variable region can also be used to produce chimeric antibodies with human variable region by means of antibody discovery.

Those skilled in the art can easily perceive other aspects and advantages of the present application from the following detailed description. In the following detailed description, only exemplary embodiments of the present application are shown and described. It will be recognized by those skilled in the art that changes can be made to the disclosed specific embodiments by those skilled in the art based on the disclosure of the present application without departing from the spirit and scope of the present application. Accordingly, correspondingly, the drawings and descriptions in the specification of the present application are only exemplary rather than restrictive.

### BRIEF DESCRIPTION OF THE DRAWINGS

Specific features of the invention to which the present application pertains are shown in the appended claims. The features and advantages of the invention to which the present application pertains can be better understood by referring to the exemplary embodiments and drawings described in detail below. A brief description of the attached drawings is as follows:
Fig. 1 shows the Cpoint and LD targeting and the modification of the C region in the heavy chain Igh constant region.
Fig. 2 schematically shows the insertion of the human heavy chain variable region, including targeting insertion and resistance marker deletion.
Fig. 3 shows the Cpoint and LD targeting and the modification of the C region in the light chain Igk constant region.
Fig. 4 schematically shows the insertion of the human light chain variable region, including targeting insertion and resistance marker deletion.
Fig. 5 schematically shows the insertion of the heavy chain constant region targeting vector, including LD targeting and constant region targeting.
Fig. 6 shows the PCR identification results of LD targeting.
Fig. 7 shows the PCR identification results of vector targeting.
Fig. 8 shows the PCR identification results of humanized HcAbs mice.
Fig. 9 shows the sequencing comparison results of PCR products for CH1 identification in the genomic DNA.
Fig. 10 shows the sequencing comparison results of PCR products for CH1 identification in IgG-mRNA.
Fig. 11 shows the test results of the utilization of IGH insertion gene.
Fig. 12 shows that the proportion of lymphocytes and myeloid cells in the spleen is normal.
Fig. 13 shows the titer detection in humanized HcAbs mice and control mice by ELISA.
Fig. 14 shows the activity detection of humanized heavy-chain only antibody against human CD98hc by ELISA.
Fig. 15 shows an exemplary modification method for changing CH1 position in the present application.

### DETAILED DESCRIPTION

The implementations of the present application will be described by way of specific embodiments, and those skilled in the art can easily understand other advantages and effects of the present application from the disclosure in the present specification.

### Definitions of terms

In the present application, the term "non-human animal" generally refers to all non-human vertebrates, for example mammals and non-mammals, such as non-human primates, rodents, rabbits, camels, sheep, dogs, cats, horses, cows, birds, amphibians and reptiles. For example, the non-human animal can be a rat, or a mouse.

In the present application, the term "CH1" usually refers to an exon encoding the first domain of the immunoglobulin heavy chain constant region IGHC or an encoded peptide fragment that located before the hinge region and specifically in the 3/4 or 4/5 region of the antibody heavy chain near the C end, and having an Ig allotype genetic marker. The term includes intron, exon, CH1 full-length gene, and CH1 functional fragment. For the position of the CH1 exon in IGHC, please refer to IMGT/GENE-DB.

In the present application, the term "CH2" usually refers to an exon encoding the second domain of the immunoglobulin heavy chain constant region or an encoded peptide fragment. The CH2 domain has a complement C1q binding site, and can participate in the activation of the typical complement pathway, thus enhancing the immune response. The term includes intron, exon, CH2 full-length gene, and CH2 functional fragment. For the position of the CH2 exon in IGHC, please refer to IMGT/GENE-DB.

In the present application, the term "CH3" usually refers to an exon encoding the third domain of the immunoglobulin heavy chain constant region or an encoded peptide fragment. For IgG antibodies, the CH3 domain can bind to Fc-gamma receptor (FcγR) on the surface of a variety of immune cells, and mediate immune cells to produce various biological effects, such as antibody-dependent cell-mediated cytotoxicity (ADCC) and antibody-dependent cell-mediated phagocytosis (ADCP). The term includes intron, exon, CH3 full-length gene, and CH4 functional fragment. For the position of the CH4 exon in IGHC, please refer to IMGT/GENE-DB.

In the present application, the term "H" usually refers to an exon encoding the immunoglobulin hinge region or an encoded peptide fragment that is located between CH1 and CH2 domains, is proline-rich and thus stretchable and bendable, and can change the distance between the two arms of the "Y" shape, to promote the simultaneous binding of the two arms to two identical epitopes. It also promotes the exposure of the complement binding site on the immunoglobulin molecule, for binding with the complement C1q to activate the complement. The hinge regions of the five types of immunoglobulins and their subclasses are different. There is a hinge region between the heavy chain CH1 and CH2 of IgG, IgA and IgD. The hinge region of IgG1, IgG2, IgG4 and IgA is short, and consists of only 10 more amino acid residues. In contrast, the hinge region of IgG3 and IgD is long, and contains about 60 more amino acid residues. The heavy chains of IgM and IgE have no hinge region. In addition, the hinge region is sensitive to papain and pepsin. After enzymatic hydrolysis, immunoglobulin can be broken into several different fragments from this region. The term includes intron, exon, H full-length gene, and H functional fragment. For the position of the H exon in IGHC, please refer to IMGT/GENE-DB.

In the present application, the term "M1" generally refers to an exon encoding the transmembrane domain of the immunoglobulin constant region, so that the antibody can be expressed on the cell membrane or secreted extracellularly. M1 is a transmembrane region close to the CH3 domain, which is involved in the transmembrane transport and localization of the antibody molecule on the cell membrane. The M1 domain is very important for the correct folding and stability, and the transport and distribution of the antibody molecule in the body. It ensures that the antibody can be effectively transported outside the cell to exert its immune functions. The M1 domain usually contains hydrophobic amino acids, which are structurally helpful for the antibody molecules to cross the phospholipid bilayer of the cell membrane. The term includes intron, exon, M1 full-length gene, and M1 functional fragment. For the position of the M1 exon in IGHC, please refer to IMGT/GENE-DB.

In the present application, the term "M2" generally refers to an exon encoding the transmembrane domain of the immunoglobulin constant region, so that the antibody can be expressed on the cell membrane or secreted extracellularly. M2 is a transmembrane region located downstream of M1, which is also involved in the transmembrane transport and localization of the antibody. The term includes intron, exon, M2 full-length gene, and M2 functional fragment. For the position of the M2 exon in IGHC, please refer to IMGT/GENE-DB.

In the present application, the term "L" generally refers to an element encoding a linker that is located between the M2 and CH1 domains and can connect the M2 and CH1 in the cells.

In the present application, the term "2A peptide" usually refers to a viral oligopeptide with 18-22 amino acids (aa) in length, which mediates the "self-cleavage" of the polypeptide in the process of translation in the eukaryotic cells. The mechanism of "self-cleavage" mediated by 2A is that the ribosome skips the glycyl-prolyl peptide bond at the C end of the 2A peptide, thus forming the upstream and downstream proteins of 2A. "2A" refers to a specific region in the virus genome, and different viral 2As are usually named after the viruses from which they are derived. Common 2A peptides include F2A, E2A, P2A and T2A.

In the present application, the term "constant region" generally refers to a sum of domains of an antibody except variable regions. The constant region does not directly involve the binding of an antigen, but shows different effector functions. Depending on the amino acid sequences of the constant regions of their heavy chains, the antibodies are classified as the following categories: IgA, IgD, IgE, IgG and IgM, and some of them can be further classified as categories IgG1, IgG2 and IgG3, as well as IgG4, IgA1 and IgA2. The heavy chain constant regions corresponding to different categories of antibodies are referred to as α, δ, ε, γ and µ, respectively. The light chain constant regions found in all of 5 categories of antibodies are referred to as κ (kappa) and λ (lambda). The genes encoding mouse (Mus musculus) constant regions can comprise IGHA, IGHD, IGHE, IGHG1, IGHG2A, IGHG2B, IGHG2C, IGHG3 or IGHM. Information about mouse constant region loci can see IMGT Repertoire:https://www.imgt.org/IMGTrepertoire/index.php?section=LocusGenes&repertoire=ge netable&species=Mus_musculus&group=IGHC.

In the present application, the term "locus" generally refers to a specific region along a chromosome or a DNA sequence. Depending on the contexts, the locus can be a specific sequence of a gene, a marker, a chromosome band, or one or more nucleotides. In the present application, when it comes to the immunoglobulin locus, the locus comprises a genetic element or a set of related genetic elements whose cells can be used for expressing the information of an immunoglobulin peptide. As to a locus that is not rearranged, the genetic element can be assembled by a B cell precursor to form a gene encoding the immunoglobulin peptide. As to a locus that is rearranged, the gene encoding the immunoglobulin peptide is contained in the locus.

In the present application, the term "antibody" generally refers to a support or skeleton portion comprising an intact antibody or an antigen binding fragment thereof, and optionally allowing a portion binding to the antigen to adopt a conformation promoting the binding of the antibody to the antigen. The examples of the antibody include but are not limited to a monoclonal antibody, a polyclonal antibody, Fab, Fab', F(ab)2, a Fv fragment, F(ab')2, scFv, di-scFv and/or dAb, an immunoconjugate, a multi-specific antibody (for example a bispecific antibody), an antibody fragment, an antibody derivative, an antibody analogue or a fusion protein and the like, as long as they show required antigen binding activity. The term also includes a genetically engineered antibody such as a chimeric antibody (for example a humanized mouse antibody), a humanized antibody, a fully human antibody and a heterologous covalent antibody (for example a bispecific antibody).

In the present application, the term "chimeric antibody" generally refers to an antibody comprising sequences present in at least two different antibodies (for example, antibodies derived from two different mammal species, for example human and mouse antibodies). The non-limiting examples of the chimeric antibody are antibodies comprising variable domain sequences (for example, all or a part of light chain variable domain and/or heavy chain variable domain sequences) of a human antibody and constant domains of a non-human antibody.

In the present application, "immunoglobulin" generally refers to a protein essentially consisting of one or more polypeptides encoded by immunoglobulin genes. The recognized human immunoglobulin genes comprise κ, λ, α (IgA1 and IgA2), γ (IgG1, IgG2, IgG3, and IgG4), δ, ε and µ constant region genes, as well as numerous immunoglobulin variable region genes. The NH2- terminal (about 110 amino acids) of a full-length immunoglobulin "light chain" (about 25 KD and 214 amino acids) is encoded by variable region genes, and the COOH- terminal is encoded by κ or λ constant region genes. The full-length immunoglobulin "heavy chain" (about 50 KD and 446 amino acids) is encoded similarly by variable region gene (about 116 amino acids) and one of the above other constant region genes such as γ (encoding about 330 amino acids). The term "immunoglobulin" comprises immunoglobulins of CDR from human or non-human sources. The structure of the immunoglobulin can be human, humanized or non-human, for example a mouse structure with reduced antigenicity in a human body via modification, or a synthesized structure for example a consensus sequence.

In the present application, the term "variable region" generally refers to a region where an antibody molecule binds to a specific antigen. It consists of antigen binding sites of a heavy chain and a light chain. The variable regions are different between different B cell immunoglobulins, but are the same between all the immunoglobulins produced by the same B cell. The diversity of the variable region is generated by gene recombination of variable region genes during the B cell maturation. This process is a rearrangement process which generates lots of diversity capable of binding to any given antigens, and therefore an immune system recognizes and neutralizes a large amount of antigenic burdens caused by exogenous and pathogenic structures. Therefore, an antibody library is composed of rich immunoglobulins having different V regions, but the immunoglobulins have the same Fc portion.

In the present application, the term "endogeneity" generally refers to any substance derived from or within organisms, cells, tissues, or systems. For example, an endogenous gene, i.e., a gene naturally occurring in a non-human animal, is different from an exogenous gene that is from other animals, and introduced through transgenosis.

In the present application, the terms "upstream" and "downstream" are generally descriptions of relative positions between genes in a chromosome, with reference to the chromosome, or a given gene. In the present application, to better conclude modification common points of heavy chain and light chain loci, with reference to natural immunoglobulin locus position of a non-human animal host and its immunoglobulin transcription direction (i.e., as shown in NCBI database), for example, for gene A, if the position of the gene A is the rear of the coding terminal of the immunoglobulin locus relative to the host immunoglobulin locus, it is considered that A is downstream and the host immunoglobulin locus is upstream.

In the present application, the term "transcription direction" generally refers to a gene coding direction where genes finally form a functional protein, i.e., a direction from a sequence of a coding initiation codon (such as ATG) to a sequence of a coding termination codon (such as TAG, TAA and TGA).

### Detailed Description

The present application relates to a genetically modified non-human animal and cell that retain the CH1 exon and can produce an antibody without the CH1 domain. In the present disclosure, the research work carried out with mice is just an example. Unless otherwise specified, the reference to mice includes all non-human mammals, and mice are the preferred non-human mammals.

The genetically modified non-human animal of the present application can be used to produce a wide variety of heavy chain antibodies. The genetic modifications described herein can be produced in, for example, any suitable mouse strain. The mouse strain can have any genetic background suitable for producing the selected heavy chain antibody.

In one aspect, the non-human animal or cell involved in the present application has a genome with an immunoglobulin heavy chain constant region gene including the following exons: CH2, CH3 and CH1; and the exon order is CH2, CH3 and CH1 in sequence.

In some embodiments, the immunoglobulin heavy chain constant region gene includes the following exons: H, CH2, CH3, M1, M2, L and CH1, and the exon order is H, CH2, CH3, M1, M2, L, and CH1 in sequence.

In some embodiments, L is a linker in a protein, and generally includes a flexible linker, a rigid linker and a cleavable linker.

In some embodiments, L is F2A, E2A, P2A or T2A.

In some embodiments, the modified immunoglobulin heavy chain constant region in the non-human animal is IgM, IgD, IgA, IgE, IgG3, IgG1, IgG2A, IgG2B and/or IgG2C.

In some embodiments, the endogenous immunoglobulin light chain variable region gene of the non-human animal is not modified or deleted.

In some embodiments, the endogenous immunoglobulin heavy chain variable region gene of the non-human animal is not modified or deleted.

Moreover, the non-human animal or cell involved in the present application includes one or multiple human IGHV region(s), one or multiple human IGHD region(s) and/or one or multiple human IGHJ region(s) operably linked downstream of the heavy chain constant region gene locus of the non-human animal. In some embodiments, after the human IGHV region, human IGHD region and human IGHJ region are inserted, the transcription direction of the coding region is reverse to that of the coding region of the original endogenous heavy chain VDJ gene in the host. The human IGHV region, human IGHD region and human IGHJ region are operably linked, and can undergo VDJ recombination.

In some embodiments, the endogenous heavy chain constant region gene locus in the animal is totally or partly modified, so that the transcription direction is totally or partly reverse to that of the endogenous heavy chain variable region gene.

Moreover, the non-human animal or cell involved in the present application includes one or multiple human light chain (e.g. IGK) V region and/or one or multiple human light chain (e.g. IGK) J region downstream of the coding region in the light chain gene locus of the host non-human animal. In some embodiments, after the human light chain (e.g. IGK) V region and human light chain (e.g. IGK) J region are inserted, the transcription direction of the coding region is reverse to that of the coding region of the corresponding original endogenous light chain VJ gene in the host. The human IGKV region and human IGKJ region are operably linked, and can undergo VJ recombination.

In some embodiments, the endogenous light chain constant region gene locus in the animal is totally or partly modified, so that the transcription direction is totally or partly reverse to that of the corresponding endogenous light chain variable region gene.

In one aspect, the human immunoglobulin heavy chain VDJ region gene located downstream of the coding region of the heavy chain gene locus in the host non-human animal contains all V, D and J regions of human heavy chain and some or all intervening sequences arranged reversely in a manner of germline.

Moreover, the human immunoglobulin heavy chain VDJ region gene located downstream of the coding region of the heavy chain gene locus in the host non-human animal is reversely arranged and can be operably linked to the modified constant region of the host, so as to undergo VDJ recombination smoothly and express a chimeric antibody with the functional human variable region.

Moreover, the reversely arranged human immunoglobulin gene located downstream of the coding region of the heavy chain gene locus in the host non-human animal can be constitutively expressed with the constant region of different antibody subtypes of the host, for smooth immunoglobulin isotype switching in B cells.

In one aspect, the human immunoglobulin light chain VJ region gene located downstream of the coding region of the light chain gene locus in the host non-human animal contains all V and J regions of human light chain and some or all intervening sequences arranged reversely in a manner of germline.

In another aspect, the human immunoglobulin light chain VJ region gene located downstream of the coding region of the light chain gene locus in the host non-human animal is reversely arranged and can be operably linked to the modified constant region of the host, so as to undergo VJ recombination smoothly and express a chimeric antibody with the functional human variable region.

In one aspect, the inserted human immunoglobulin variable region gene DNA contains 10%-100%, such as 60% or higher, 70% or higher, 80% or higher, or 90% or higher, of the coding region sequence of reverse human heavy chain variable (V) gene and the coding region of complete human heavy chain variable region gene.

In one aspect, the inserted human immunoglobulin variable region gene DNA contains 50%-100%, such as 60% or higher, 70% or higher, 80% or higher, or 90% or higher, of the coding region of reverse human heavy chain D region gene and the coding region of complete human D region gene.

In one aspect, the inserted human immunoglobulin variable region gene DNA contains 50%-100%, such as 60% or higher, 70% or higher, 80% or higher, or 90% or higher, of the coding region of reverse human heavy chain J region gene and the coding region of complete human J region gene.

In one aspect, the inserted human immunoglobulin heavy chain variable region gene DNA has a length of about or at least 10 kb, 20 kb, 30 kb, 40 kb, 50 kb, 60 kb, 70 kb, 80 kb, 90 kb, 100 kb, 200 kb, 300 kb, 400 kb, 500 kb, 600 kb, 700 kb, 800 kb, 900 kb, 1000 kb, 1500 kb, 2000 kb, 2500 kb, 3000 kb or 3500 kb.

In one aspect, the inserted human immunoglobulin variable region gene DNA contains 15%-100%, such as 60% or higher, 70% or higher, 80% or higher, or 90% or higher, of the coding region of reverse human light chain V region gene and the coding region of complete human V region gene.

In one aspect, the inserted human immunoglobulin variable region gene DNA contains 50%-100%, such as 60% or higher, 70% or higher, 80% or higher, or 90% or higher, of the coding region of reverse human light chain J region gene and the coding region of complete human light chain J region gene.

In one aspect, the inserted human immunoglobulin light chain variable region gene DNA has a length of about or at least 10 kb, 20 kb, 30 kb, 40 kb, 50 kb, 60 kb, 70 kb, 80 kb, 90 kb, 100 kb, 200 kb, 300 kb, 400 kb, 500 kb, 600 kb, 700 kb, 800 kb, 900 kb, 1000 kb, 1500 kb, 2000 kb, 2500 kb, 3000 kb or 3500 kb.

For the information of human heavy chain V region, D region and J region, and light chain V region and J region, see IMGT Repertoire: https://www.imgt.org/IMGTrepertoire/LocusGenes/.

Optionally, the non-human animal may contain only the human heavy chain variable region gene located downstream of the coding region of the heavy chain gene locus of the host non-human animal, or only the human light chain variable region gene located downstream of the coding region of the light chain gene locus of the host non-human animal, or both of them.

Optionally, the endogenous immunoglobulin heavy chain variable region (V, D, and J regions) gene of the non-human animal is not modified or deleted, to avoid the influence on the expression or regulation of some genes in this variable region of the host. Moreover, the endogenous immunoglobulin heavy chain variable region is far away from the inserted human immunoglobulin heavy chain variable region, and they are spaced apart by the endogenous immunoglobulin heavy chain constant region coding sequence. The distance in base length between them is between 169 Kbp and 240 Kbp, and preferably 202 Kbp. In this way, the endogenous variable region is prevented from incorporation into the final chimeric immunoglobulin coding gene recombination to some extent.

Optionally, the endogenous immunoglobulin light chain variable region (V and J regions) gene of the non-human animal is not modified or deleted. to avoid the influence on the expression or regulation of some genes in this variable region of the host. Moreover, the endogenous immunoglobulin light chain variable region is far away from the inserted human immunoglobulin light chain variable region, and they are spaced apart by the endogenous immunoglobulin light chain constant region coding sequence. The distance in base length between them is between 4 Kbp and 42 Kbp, and preferably 5 Kbp. In this way, the endogenous variable region is prevented from incorporation into the final chimeric immunoglobulin coding gene recombination to some extent.

The present application discloses a method for constructing, in a non-human animal (for example, mice), an immunoglobulin heavy chain constant region gene having an exon order of: CH2, CH3 and CH1 in sequence. In the present application, the research work carried out with mice is just an example. Unless otherwise specified, the reference to mice includes all non-human animals, and mice are the preferred non-human animals.

In one aspect, the DNA sequence having an exon order of CH2, CH3 and CH1 in sequence is inserted into a region targeted between exon IGHJ4 and Tmem121 gene locus of mouse chromosome 12 by site-directed recombination. In an aspect, it is inserted in mouse chromosome 12 between coordinates 113,191,291-113,391,844.

In one aspect, the endogenous immunoglobulin heavy chain constant region IGHG gene of the non-human animal is replaced by the DNA sequence having an exon order of CH2, CH3 and CH1 in sequence.

In one aspect, the inserted immunoglobulin heavy chain constant region CH1 domain contains 50-100%, such as 60% or higher, 70% or higher, 80% or higher, or 90% or higher, of the coding region sequence of the immunoglobulin heavy chain constant region CH1 domain and the coding region of complete human heavy chain variable region gene.

In some embodiments, the non-human embryos are mouse embryos or rat embryos, and the donor cells are mouse ES cells or rat ES cells, respectively.

In some embodiments, the site-specific recombination includes homologous recombination, nucleic acid cleaving enzyme-mediated recombination method (such as CRISPR/Cas9, etc.), and site-specific recombinase-mediated recombination. It involves the combined use of one or more methods. The insertion or replacement can be achieved by common gene editing and targeting methods, such as homologous recombination or nucleic acid cleaving enzyme-mediated recombination (such as CRISPR/Cas9, etc.). The modification can also be achieved by a nuclease, and related technologies are known in the prior art.

In some embodiments, CH1 in the endogenous immunoglobulin heavy chain constant region IGHG gene of the non-human animal cell is cleaved off, and then L and CH1 are inserted after CH3, so as to have an exon order of CH2, CH3 and CH1. For example, L+CH1 can be inserted after M2. Aft screening and identification of the obtained modified cells, the cells are injected into early embryos to prepare an animal (for example, mice). Subsequent breeding can be carried out to obtain animals containing the non-humanized immunoglobulin gene locus.

In some embodiments, the endogenous immunoglobulin heavy chain constant region IGHG gene of the non-human animal cells is overall replaced by the DNA sequence having an exon order of CH2, CH3 and CH1 in sequence. Aft screening and identification of the obtained modified cells, the cells are injected into early embryos to prepare an animal (for example, mice). Subsequent breeding can be carried out to obtain animals containing the non-humanized immunoglobulin gene locus.

In another aspect, the present application discloses a method for constructing a non-human animal (for example, a mouse) having a functional human immunoglobulin variable region gene.

In some embodiments, the immunoglobulin variable region gene of the non-human animal is modified or deleted, then CH1 in the endogenous immunoglobulin heavy chain constant region IGHG gene of the non-human animal is cleaved off, and L and CH1 are inserted after CH3, so as to have an exon order of CH2, CH3 and CH1. Aft screening and identification of the obtained modified cells, the cells are injected into early embryos to prepare a chimeric animal (for example, mouse). Subsequent breeding can be carried out to obtain animals containing complete humanized immunoglobulin gene locus.

In some embodiments, the immunoglobulin variable region gene of the non-human animal is modified or deleted, and then the endogenous immunoglobulin heavy chain constant region IGHG gene of the non-human animal cells is overall replaced by the DNA sequence having an exon order of CH2, CH3 and CH1. Aft screening and identification of the obtained modified cells, the cells are injected into early embryos to prepare a chimeric animal (for example, mouse). Subsequent breeding can be carried out to obtain animals containing complete humanized immunoglobulin gene locus.

In some embodiments, CH1 in the endogenous immunoglobulin heavy chain constant region IGHG gene of the non-human animal cell is cleaved off, and L and CH1 are inserted after CH3, so as to have an exon order of CH2, CH3 and CH1. Then, the immunoglobulin variable region gene of the non-human animal is modified or deleted. After screening and identification of the obtained modified cells, the cells are injected into early embryos to prepare a chimeric animal (for example, mouse). Subsequent breeding can be carried out to obtain animals containing complete humanized immunoglobulin gene locus.

In some embodiments, the endogenous immunoglobulin heavy chain constant region IGHG gene of the non-human animal cells is overall replaced by the DNA sequence having an exon order of CH2, CH3 and CH1. Then, the immunoglobulin variable region gene of the non-human animal is modified or deleted. After screening and identification of the obtained modified cells, the cells are injected into early embryos to prepare a chimeric animal (for example, mouse). Subsequent breeding can be carried out to obtain animals containing complete humanized immunoglobulin gene locus.

In some embodiments, the transcription direction of the modified human variable region gene is reverse to that of the endogenous variable region gene of the non-human animal. In some embodiments, the transcription direction of the modified endogenous constant region gene is reverse to that of the endogenous variable region gene.

In some embodiments, the modified human variable region gene is inserted downstream of (after) the endogenous immunoglobulin gene locus in the mouse cells, then a modified short exogenous recombinase binding site is introduced into the endogenous constant region in the mouse cells, and the endogenous constant region in the mouse cells is partially or completely reversed by introducing a recombinase. Then CH1 in the endogenous immunoglobulin constant region IGHG gene in the mouse cells is cleaved off, and L and CH1 are inserted after CH3, so as to have an exon order of CH2, CH3, L, and CH1. After screening and identification of the obtained modified cells, the cells are injected into early embryos to prepare a chimeric animal (for example, mouse). Subsequent breeding can be carried out to obtain animals containing complete humanized immunoglobulin gene locus.

In some embodiments, the modified human variable region gene is inserted downstream of (after) the endogenous immunoglobulin gene locus in the mouse cells, then a modified short exogenous recombinase binding site is introduced into the endogenous constant region of the mouse cells, the endogenous constant region in the mouse cells is partially or completely reversed by introducing a recombinase. Then the endogenous immunoglobulin heavy chain constant region IGHG gene in the mouse cells is overall replaced by the DNA sequence having CH2, CH3 and CH1, so as to have an exon order of CH2, CH3 and CH1. After screening and identification of the obtained modified cells, the cells are injected into early embryos to prepare a chimeric animal (for example, mouse). Subsequent breeding can be carried out to obtain animals containing complete humanized immunoglobulin gene locus.

In some embodiments, a modified short exogenous recombinase binding site is introduced into the endogenous constant region of the mouse cells, and the endogenous constant region in the mouse cells is partially or completely reversed by introducing a recombinase. Then, the modified human variable region gene is inserted downstream of (after) the endogenous immunoglobulin gene locus in the mouse cells. Then CH1 in the endogenous immunoglobulin heavy chain constant region IGHG gene in the mouse cells is cleaved off, and L and CH1 are inserted after CH3, so as to have an exon order of CH2, CH3, L, and CH1. After screening and identification of the obtained modified cells, the cells are injected into an early embryo of an animal to prepare a chimeric animal (for example, a mouse). Subsequent breeding can be carried out to obtain animals containing complete humanized immunoglobulin gene locus.

In some embodiments, a modified short exogenous recombinase binding site is introduced into the endogenous constant region of the mouse cells, and the endogenous constant region in the mouse cells is partially or completely reversed by introducing a recombinase. Then, the modified human variable region gene is inserted downstream of (after) the endogenous immunoglobulin gene locus in the mouse cells. Then the endogenous immunoglobulin heavy chain constant region IGHG gene in the mouse cells is overall replaced by the DNA sequence having CH2, CH3 and CH1, so as to have an exon order of CH2, CH3, L, and CH1. After screening and identification of the obtained modified cells, the cells are injected into an early embryo of an animal to prepare a chimeric animal (for example, a mouse). Subsequent breeding can be carried out to obtain animals containing complete humanized immunoglobulin gene locus.

In some embodiments, the modified human variable region gene is inserted downstream of (after) the endogenous immunoglobulin gene locus in the mouse cells, and then the endogenous immunoglobulin heavy chain constant region IGHG gene in the mouse cells is overall replaced by the DNA sequence having CH2, CH3 and CH1, so as to have an exon order of CH2, CH3, L, and CH1. After screening and identification of the obtained modified cells, the cells are injected into an early embryo of an animal to prepare a chimeric animal (for example, a mouse). Subsequent breeding can be carried out to obtain animals containing complete humanized immunoglobulin gene locus.

In some embodiments, the endogenous immunoglobulin heavy chain constant region IGHG gene in the mouse cells is overall replaced by the DNA sequence having CH2, CH3 and CH1, so as to have an exon order of CH2, CH3, L and CH1. The modified human variable region gene is inserted downstream of (after) the endogenous immunoglobulin gene locus in the mouse cells. For example, the transcription direction of the modified constant region is reverse to that before modification. After screening and identification of the obtained modified cells, the cells are injected into an early embryo of an animal to prepare a chimeric animal (for example, mouse). Subsequent breeding can be carried out to obtain animals containing complete humanized immunoglobulin gene locus.

In one aspect, the insertion of the human immunoglobulin heavy chain variable region gene DNA is targeted between the gene Tmem121 and gene Igha downstream of the IgH gene locus on mouse chromosome 12 by site-directed recombination. In an aspect, it is inserted between coordinates 113,149,523 and 113,223,857 on mouse chromosome 12, and suitably at coordinate 113,190,256.

In one aspect, the insertion of the human immunoglobulin light chain variable region gene DNA such as human light chain κVJ is targeted between the lgkc and Rpia genes downstream of the IgK gene locus on mouse chromosome 6 by site-directed recombination. In an aspect, it is inserted between coordinates 70,703,738 and 70,742,704 on mouse chromosome 6 and suitably at position 70,706,267, or at an equivalent position with respect to the λ locus on mouse chromosome 16. All coordinates are based on NCBI database GRCm39.

Optionally, in some embodiments, only the human heavy chain variable region gene is introduced downstream of the coding region of the heavy chain gene locus of the host non-human animal, or only the human light chain variable region gene is introduced downstream of the coding region of the light chain gene locus of the host non-human animal, or both of them are introduced.

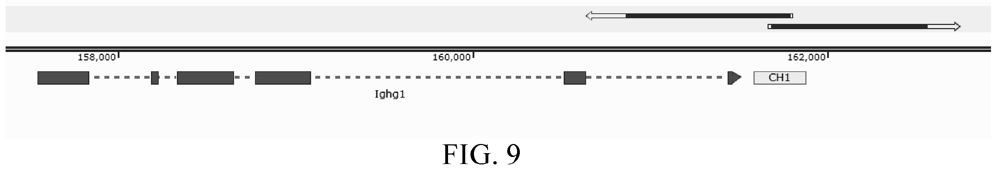

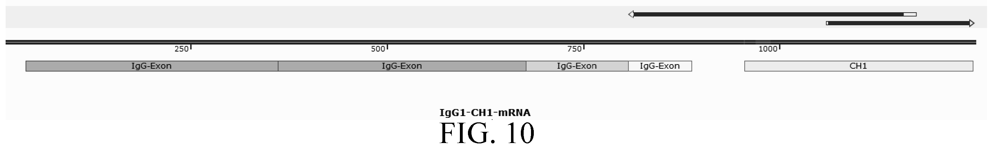

In an embodiment, the whole human variable region gene is inserted into the mouse gene locus by gene targeting technology in the present application, and the heavy-chain only model is obtained by changing the position of IgG-CH1 exon on the original IgG gene, instead of knocking out CH1. The non-human animal model of the present application still has CH1 functional domain on the gene (Figs. 9 and 10).

The technical effects achievable by the embodiments of the present application are as follows. For example, the non-human animal of the present application contains complete human heavy chain variable region VDJ gene and can express the humanized heavy-chain only antibody, thus reducing the cost and difficulty of screening human heavy-chain only antibodies. The non-human animal of the present application retains the CH1 nucleic acid sequence and the endogenous heavy chain regulatory element, thus greatly reducing the adverse effects caused by gene or exon deletion.

Without being bound by any theory, the following examples are only for illustrating the non-human animal, preparation method and use of the present application, and not intended to limit the scope of the present application.

### Examples

### Example 1: Mouse ES cells inserted with human variable region

The ES cell line of BALB/c mice was obtained by independent isolation, which was used as the basic material for subsequent targeting.

By using ES targeting technology well known in the art, a vector construct 1 (hereinafter referred to as Cpoint, construct for introducing human variable region insertion) was respectively knocked into a position downstream of the mouse-derived IGH and IGK constant region gene loci in the ES cells (a region between Igha gene and Tmem121 gene, specifically IGH chr12:113,190,256; and a region between Igkc and Rpia genes, specifically IGK chr6:70,706,267. NCBI database GRCm39).

After the correct ES cell line was obtained, a vector construct 2 (hereinafter referred to as LD, which was used to cooperate with Cpoint) was respectively knocked into a position upstream of the mouse-derived IGH and IGK constant regions (a region between IgH J4 exon and Cµ gene locus, specifically IGH chr12: 113,391,844; and a region between Igk J5 exon and Igkc gene, specifically IGK chr6: 70,701,630. NCBI database GRCm39).

The above-mentioned positive cell line was transfected with Cre expression plasmid, and the mouse C region was reversed by using the Cre/loxP recombination mechanism (Zheng, B et al. "Engineering a mouse balancer chromosome." Nature genetics vol. 22,4 (1999): 375-8. doi:10.1038/11949). The positive cell line was further transfected with a transposase expression plasmid, and the screening marker fragment was deleted by using the transposition mechanism (Transposase/Transposon system) (Maragathavally, K. J., et al. "Chimeric Mos1 and piggyBac transposases result in site-directed integration." The FASEB journal 20.11 (2006): 1880-1882. Wilson, Matthew H., Craig J. Coates, and Alfred L. George. "PiggyBac transposon-mediated gene transfer in human cells." Molecular therapy 15.1 (2007): 139-145.).

By using the recombinase-mediated cassette exchange (RMCE) technology (Wallace, Helen A C et al. "Manipulating the mouse genome to engineer precise functional syntenic replacements with human sequence." Cell vol. 128,1 (2007): 197-209. doi:10.1016/j.cell.2006.11.044, Prosser, Haydn M et al. "Mosaic complementation demonstrates a regulatory role for myosin VIIa in actin dynamics of stereocilia." Molecular and cellular biology vol. 28,5 (2008): 1702-12. doi:10.1128/MCB.01282-07), vector targeting was carried out in batches. After each vector insertion, the screening marker fragment was deleted by using the transposition mechanism, until the last vector was inserted. Finally, the target human antibody variable region coding gene (VDJ) was completely inserted into the ES cell line.

The ES cells analyzed by PCR and karyotyping Q-PCR array for quality control were injected into the blastocyst cavity of mice according to the method described in "Manipulating the Mouse Embryo: A Laboratory Manual, Fourth Edition (Cold Spring Harbor Laboratory Press, 2014)", to obtain a chimeric mouse, which were bred with BALB/c mouse. After genotype identification and function verification, mice containing the human immunoglobulin heavy chain variable region gene and the human immunoglobulin light chain variable region gene were finally obtained. To obtain the function verification data more quickly, an alternative solution was to inject ES cells into mouse blastocysts (such as Rag1 gene deficient mice) that cannot undergo functional VDJ rearrangement, and the obtained chimeric mice were directly verified for the immune system function without breeding.

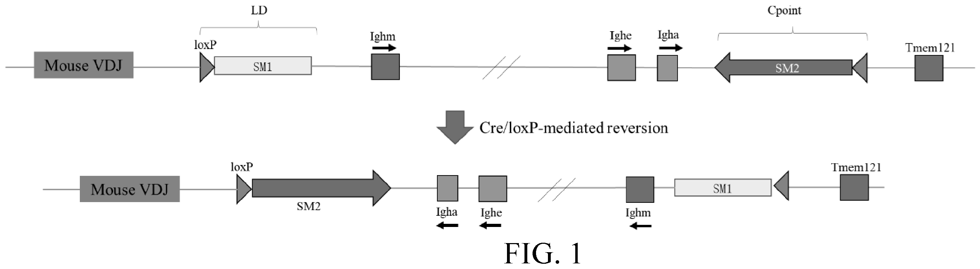

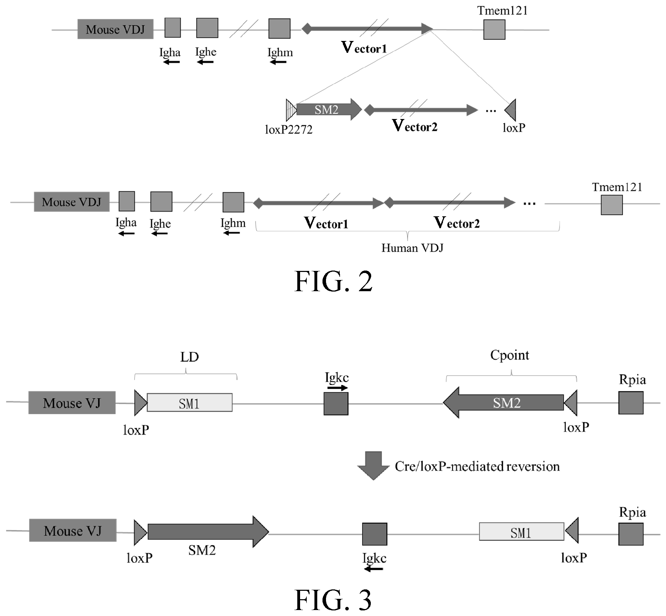

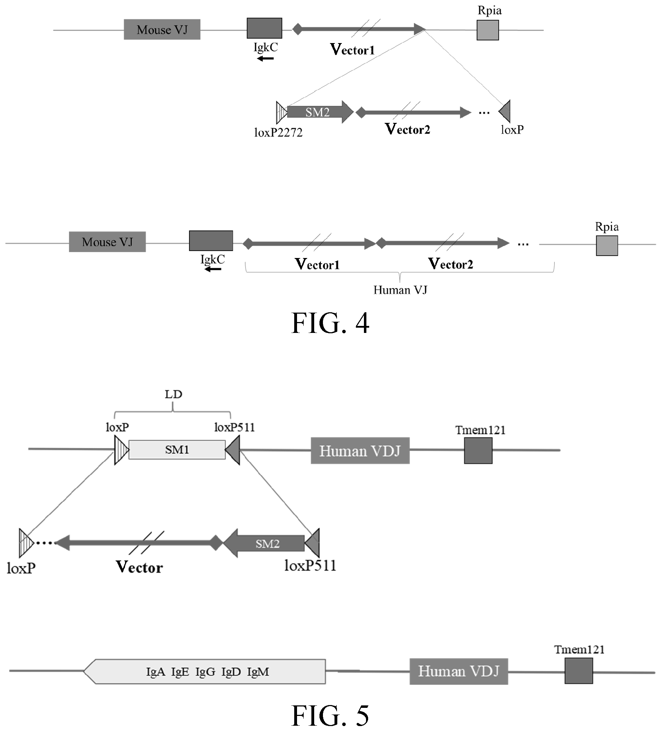

The preparation process and vector design are shown in Fig. 1- Fig. 4. Fig. 1 shows the Cpoint and LD targeting and the modification of the C region in the Igh constant region. Fig. 2 schematically shows the insertion of the human heavy chain variable region, showing IGH-vector targeting and resistance marker deletion. Fig. 3 shows the Cpoint and LD targeting and the modification of the C region in the Igk constant region. Fig. 4 schematically shows the insertion of the human light chain variable region, showing IGK-vector targeting and resistance marker deletion.

Then mouse embryonic stem cells were obtained by embryo manipulation and cell culture.

### Example 2: Construction of LD targeting vector

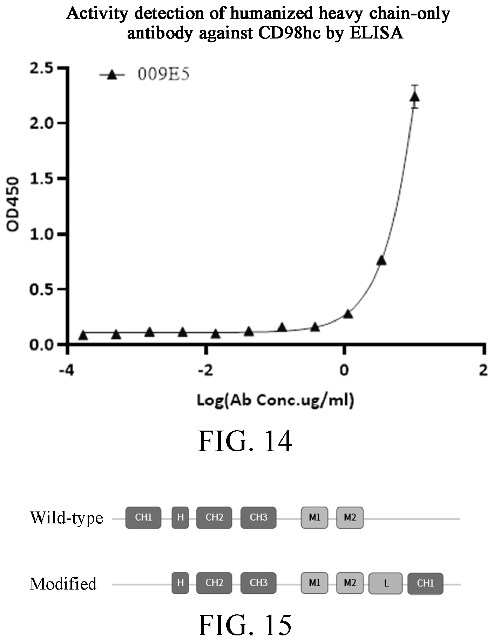

On the basis of the positive cells obtained in Example 1, by further using the ES targeting technology well known in the art and using the strategy of overall replacement (as shown in Fig. 5) in the present application, the endogenous Igh constant region was overall replaced by vector containing CH2, CH3 and CH1 constructed in vitro, so as to have an exon order of CH2, CH3 and CH1, or CH2, CH3, L, and CH1. Here, the strategy of gradual replacement of constant region CH1 could also be used. CH1 in the endogenous immunoglobulin constant region IGH gene in the mouse cells was deleted, and then CH1 was inserted after endogenous CH3 by homologous recombination, so as to have an exon order of CH2, CH3 and CH1 (as shown in Fig. 15).

In the present application, the strategy of overall replacement was used, in which LD targeting was achieved by homologous recombination, and then vector was inserted with the help of the Cre/loxP system.

The LD targeting vector consisted of homologous arms at both ends (sequences flanking the insertion site), a screening marker (SM1), and loxP and loxP511 fragments. As shown in Fig. 5, these fragments were linked to the commercial vector PMD18T by enzymatic ligation to construct the LD targeting vector, which were used to cooperate with the vector targeting vector.

By using the ES targeting technique well known in the art, the LD targeting fragment was knocked into the mouse-derived IGH constant region (the region between Ighj4 exon and Tmem121 gene locus, specifically IGH chr12: 113,191,291-113,391,844. NCBI database Mus musculus strain C57BL/6J, GRCm39) in ES cells.

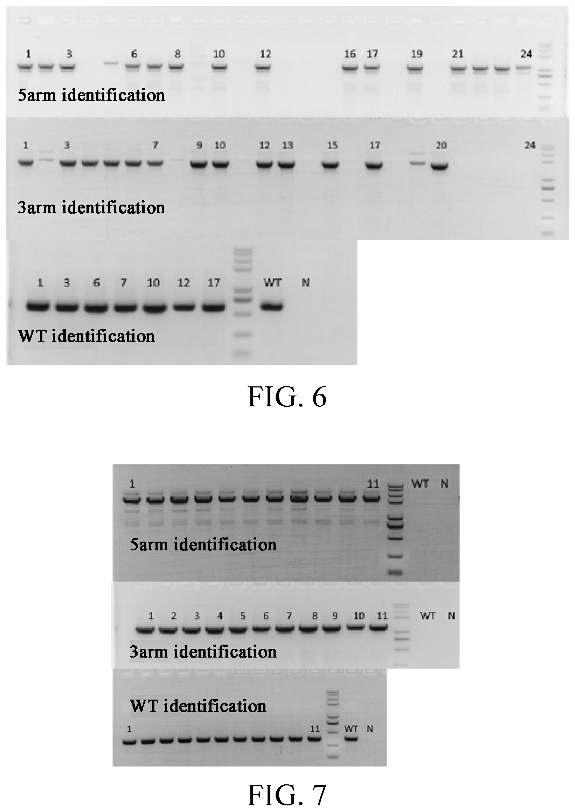

PCR identification confirmed that the LD of clones #1, 3, 6, 7, 10, 12, and 17 was targeted correctly. The LD identification scheme is shown in Table 1, and the PCR identification result is shown in Fig. 6.

**Table 1. LD identification scheme**

| **No.** | **Primer name** | **Primer sequence** | **SEQ ID NO** | **Product size** |
|---|---|---|---|---|
| ① 5 arm | H-LD-5tF1 | cgtcctgacaggggcttagg | 1 | KI=1122 bp |
| | H-LD-5tR1 | ACTTCTCGACAGACGTCGCGGT | 2 | WT=/bp |
| ② 3 arm | H-LD-3tF1 | ggctctatggcttctgaggcg | 3 | KI=1679 bp |
| | H-LD-3tR1 | gggtgtgtgcttccatctaaaat | 4 | WT=/bp |
| ③ WT | H-LD-wtF1 | GCATGATGACAGCATTATGATGGC | 5 | WT=628 bp |
| | H-LD-wtR1 | ACCAGGCACAGGGTCTACCAT | 6 | KI=/bp |

### Example 3: Construction of vector targeting vector

As shown in Fig. 5, by the Red/ET recombination technology (Rivero-Müller,Adolfo et al. "Assistedlarge fragment insertion by Red/ET-recombination(ALFIRE)--an alternative andenhanced method for large fragment recombineering." Nucleic acids researchvol.35,10(2007):e78.doi:10.1093/nar/gkm250), the endogenous CH1 sequence was recombinantly shifted to the end of the gene and the original gene sequence was replaced. Moreover, a promoter, loxP, and a screening marker (SM2) were inserted at the 5 end of the vector, and the original loxP was inserted at the 3 end of the vector, to construct the vector targeting vector.

By using the recombinase-mediated cassette exchange (RMCE) technology (Wallace,Helen AC et al. "Manipulatingthe mouse genome to engineer precise functional syntenic replacements withhuman sequence." Cell vol.128,1(2007):197-209.doi:10.1016/j.cell.2006.11.044,Prosser,Haydn M et al. "Mosaic complementation demonstrates a regulatory rolefor myosin VIIa in actin dynamics of stereocilia." Molecular and cellularbiology vol.28,5(2008):1702-12.doi:10.1128/MCB.01282-07), vector targeting was performed.

The correct vector targeting of clones #1-11 was confirmed by PCR identification. The targeting identification scheme is shown in Table 2, and the PCR identification result of vector targeting is shown in Fig. 7.

**Table 2. Vector targeting identification scheme**

| **No.** | **Primer name** | **Primer sequence** | **SEQ ID NO** | **Product size** |
|---|---|---|---|---|
| ① 5 arm | H-vector-5tF1 | cgtcctgacaggggcttagg | 1 | KI=2267 bp |
| | H-vector-5tR1 | ctgtcacagaggtggtcctga | 7 | WT=/bp |
| ② 3 arm | H-vector-3tF1 | aggtttgctgtctggacagtga | 8 | KI=1555 bp |
| | H-vector-3tR1 | ggctacatgggcattcagtaagc | 9 | WT=/bp |
| ③ WT | H-vector-wtF1 | gaatccagctgtctgataacctcac | 10 | WT=353 bp |
| | H-vector-wtR1 | ccttagttaccctctcaccgattc | 11 | KI=/bp |

Positive ES cells were injected into the blastocyst cavity of mice according to the method described in "Manipulating the Mouse Embryo: A Laboratory Manual", to obtain F0 chimeric mice, which were bred with BALB/c mice. After genotype identification and function verification, the humanized HcAbs mouse model was finally obtained.

### Example 4: Genome verification of mouse model

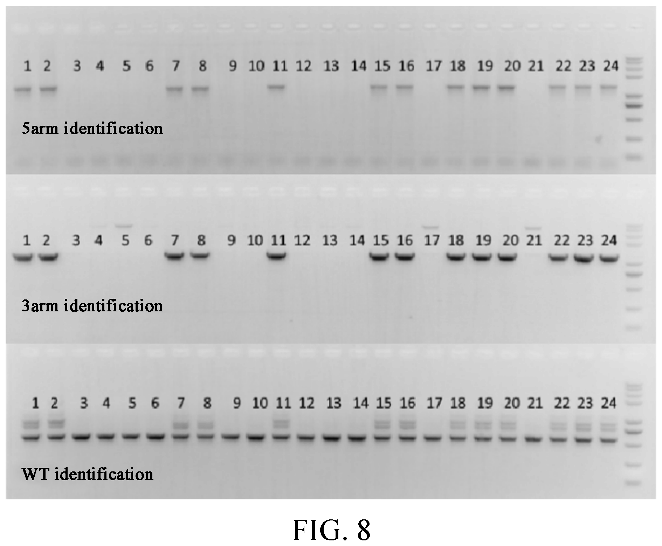

Genomic DNA of humanized HcAbs mice was extracted, and confirmed by PCR identification and sequencing that both the genome sequence and mRNA sequence of the humanized HcAbs mice have the CH1 exon sequence. The identification scheme is shown in Table 3, and the PCR identification results are shown in Fig. 8. Fig. 9 shows the sequencing comparison results of genomic PCR products. Fig. 10 shows the sequencing comparison results of PCR products in mRNA identification. The result shows that the CH1 sequence can be detected in both genome and mRNA.

**Table 3. CH1 identification scheme**

| **No.** | **Primer name** | **Primer sequence** | **SEQ ID NO** | **Product size** |
|---|---|---|---|---|
| ① 5 arm | CH1-5tF1 | ttgcaggtgacggtctcgct | 12 | KI=1321 bp |
| | CH1-5tR1 | gctgcaactggacgagacct | 13 | WT=/bp |
| ② 3 arm | CH1-3tF1 | gcccaaactaactccatggtga | 14 | KI=1246 bp |
| | CH1-3tR1 | ctgctccagctagctactgtaag | 15 | WT=/bp |
| ③ WT | CH1-wtF1 | attttgtatcttcatcctgtcccca | 16 | WT=576 bp |
| | CH1-wtR1 | ctattgctcttgtgggtctatccag | 17 | KI=/bp |

### Example 5: The humanized HcAbs mice can correctly utilize the human variable region gene

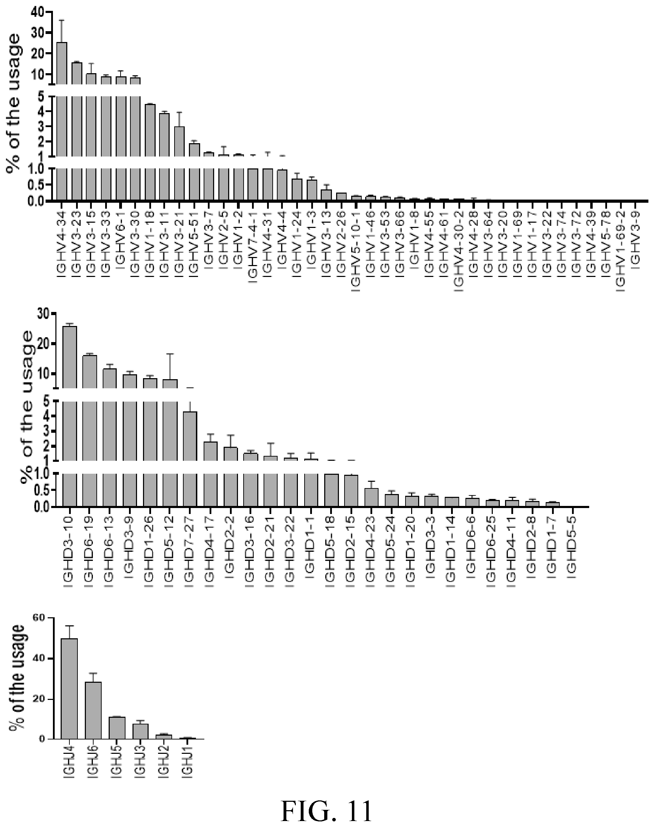

The spleen of the humanized HcAbs mice was collected, and the human *IGHV*/*D*/*J* gene usage in splenic B cells was determined by B-cell receptor (BCR) sequencing. The results of BCR sequencing are shown in Fig. 11, which proves that humanized HcAbs mice can correctly utilize the human IGH variable region gene.

### Example 6: The immune cells of humanized HcAbs mice develop normally and the proportion of B cells is normal

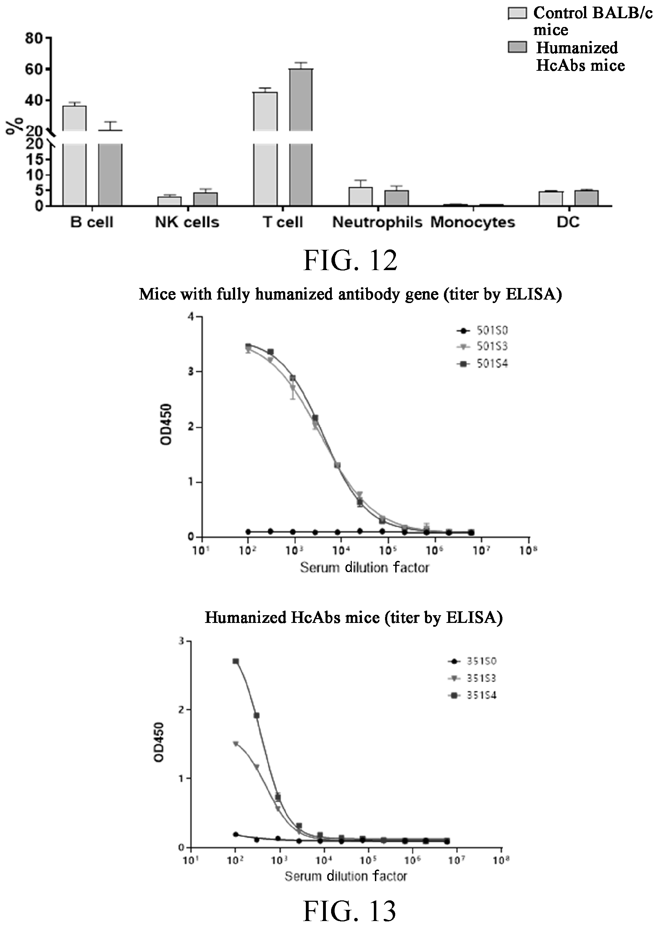

The immune-related cells in the spleen of humanized HcAbs mice were detected by flow cytometry. The results are shown in Fig. 12, which proves that the proportion of lymphocytes and myeloid cells in the spleen is normal.

### Example 7: Humanized HcAbs mice can produce a high-affinity antibody

The humanized HcAbs mice were immunized with commercial human CD98hc recombinant protein, and the titer of the antibody in the serum was detected by ELISA. The results show that the immune responses of humanized HcAbs mice and the mice with fully humanized antibody gene against the same antigen were comparable. The positive heavy-chain only antibody 009E5 was obtained by hybridoma fusion screening technology. The single heavy chain antibody was confirmed by ELISA activity assay to bind to human CD98hc protein.

The results are shown in Figs. 13 and 14, and prove that humanized HcAbs mice can produce high-affinity antibody, and the corresponding antibody has good in-vitro binding activity.

## Claims

1. A method for preparing a non-human animal, the method comprising: editing an immunoglobulin heavy chain constant region gene and changing the CH1 position in the genome of the non-human animal.

2. The method according to claim 1, wherein the immunoglobulin heavy chain constant region gene comprises the following exons: CH2, CH3 and CH1, and the **exon order** is **CH2, CH3 and CH1** in sequence.

3. The method according to any one of claims 1 and 2, wherein the CH2, CH3 and CH1 are derived from IgG and/or IgM.

4. The method according to any one of claims 1 to 3, wherein the IgG comprises IgG3, IgG1, IgG2A, IgG2B and/or IgG2C.

5. The method according to any one of claims 1 to 4, wherein the immunoglobulin heavy chain constant region gene further comprises the following exons: H, M1 and M2, and a gene L encoding a linker; and the exon order is H, CH2, CH3, M1, M2, L, and CH1 in sequence.

6. The method according to claim 5, wherein the linker is a self-cleaving peptide, and preferably a self-cleaving peptide having 18-22 amino acids in length.

7. The method according to any one of claims 5 and 6, wherein the linker is a 2A peptide, and preferably F2A, E2A, P2A and T2A.

8. The method according to any one of claims 1 to 7, wherein the immunoglobulin heavy chain constant region gene is derived from the non-human animal.

9. The method according to any one of claims 1 to 8, comprising: constructing a nucleic acid sequence, wherein the nucleic acid sequence comprises sequentially, from the 5' to 3' end, the following exons: CH2, CH3 and CH1.

10. The method according to any one of claims 1 to 9, wherein the nucleic acid sequence comprises sequentially, from the 5' to 3' end, the following exons and the gene L encoding the linker: H, CH2, CH3, M1, M2, L, and CH1.

11. The method according to any one of claims 9 and 10, comprising: inserting the nucleic acid sequence into the immunoglobulin heavy chain constant region gene locus of the non-human animal.

12. The method according to any one of claims 9 to 11, comprising: inserting the nucleic acid sequence into the immunoglobulin heavy chain constant region **IGHG** and/or IGHM gene locus of the non-human animal.

13. The method according to any one of claims 9 to 12, comprising: replacing the endogenous immunoglobulin heavy chain constant region **IGHG** and/or IGHM gene of the non-human animal by the nucleic acid sequence.

14. The method according to any one of claims 1 to 13, comprising: operably linking a human immunoglobulin heavy chain variable region gene downstream of the immunoglobulin heavy chain gene locus of the non-human animal.

15. The method according to claim 14, comprising: operably linking the gene(s) of one or multiple human heavy chain variable region V region(s), heavy chain variable region D region(s) or heavy chain variable region J region(s), or fragment(s) thereof downstream of the heavy chain constant region gene locus of the non-human animal.

16. The method according to claim 15, wherein the genes of the multiple human heavy chain variable region V regions, heavy chain variable region D regions, or heavy chain variable region J regions, or fragments thereof are directly linked.

17. The method according to any one of claims 14 to 16, wherein the non-human animal comprises an endogenous immunoglobulin heavy chain variable region gene.

18. The method according to any one of claims 16 and 17, wherein the genomic integrity of the endogenous immunoglobulin heavy chain variable region gene sequence of the non-human animal remains unchanged.

19. The method according to any one of claims 16 to 18, wherein the expression regulatory element for the endogenous immunoglobulin heavy chain variable region gene of the non-human animal is functionally unimpaired.

20. The method according to any one of claims 1 to 19, wherein the non-human animal comprises an expression regulatory element for complete endogenous immunoglobulin heavy chain variable region gene.

21. The method according to any one of claims 1 to 20, wherein the non-human animal comprises complete endogenous immunoglobulin heavy chain variable region gene.

22. The method according to any one of claims 1 to 21, wherein the non-human animal does not express the endogenous immunoglobulin heavy chain variable region.

23. The method according to any one of claims 1 to 22, wherein the endogenous immunoglobulin variable region gene of the non-human animal does not express the antibody heavy chain variable region.

24. The method according to any one of claims 1 to 23, comprising: enabling the transcription direction of the human immunoglobulin heavy chain variable region gene to be reverse to that of the endogenous immunoglobulin heavy chain variable region gene.

25. The method according to any one of claims 1 to 24, wherein the immunoglobulin heavy chain constant region gene of the non-human animal is comprised between the human immunoglobulin heavy chain variable region gene and the endogenous immunoglobulin heavy chain variable region gene in the non-human animal.

26. The method according to any one of claims 1 to 25, comprising: enabling the transcription direction of **the immunoglobulin heavy chain constant region gene** to be reverse to that of the endogenous immunoglobulin heavy chain variable region gene.

27. The method according to any one of claims 1 to 26, wherein the transcription direction of the human immunoglobulin heavy chain variable region gene is the same as that of the immunoglobulin heavy chain constant region gene in the non-human animal.

28. The method according to any one of claims 1 to 27, wherein the chromosome of the non-human animal comprises sequentially, from upstream to downstream, the endogenous immunoglobulin heavy chain variable region gene, the immunoglobulin heavy chain constant region gene, and the human immunoglobulin heavy chain variable region gene.

29. The method according to any one of claims 1 to 28, wherein the non-human animal is a rodent.

30. The method according to any one of claims 1 to 29, wherein the non-human animal is a mouse.

31. The method according to claim 30, comprising: inserting the human immunoglobulin heavy chain variable region gene between the chromosome locations chr12: 113,149,523-113,223,857 of the mouse.

32. The method according to any one of claims 30 and 31, comprising: inserting the human immunoglobulin heavy chain variable region gene at the chromosome location chr12: 113,190,256 of the mouse.

33. The method according to any one of claims 1 to 32, comprising: operably linking the human immunoglobulin heavy chain variable region gene downstream of the immunoglobulin heavy chain gene locus of the non-human animal by site-specific recombination.

34. The method according to any one of claims 1 to 33, comprising: enabling the transcription direction of the immunoglobulin heavy chain constant region gene of the non-human animal to be reverse to that of the endogenous immunoglobulin heavy chain variable region gene through genome modification.

35. A non-human animal, having a genome with an immunoglobulin heavy chain constant region gene comprising the following exons: CH2, CH3 and CH1, wherein the exon order is CH2, CH3 and CH1 in sequence.

36. The non-human animal according to claim 35, wherein the CH2, CH3 and CH1 are derived from IgG and/or IgM.

37. The non-human animal according to claim 36, wherein the IgG comprises IgG3, IgG1, IgG2A, IgG2B and/or IgG2C.

38. The non-human animal according to any one of claims 35 to 37, wherein the immunoglobulin heavy chain constant region gene further comprises the following exons: H, M1 and M2, and a gene L encoding a linker; and the exon order is H, CH2, CH3, M1, M2, L, and CH1 in sequence.

39. The non-human animal according to claim 38, wherein the linker is a self-cleaving peptide, and preferably a self-cleaving peptide having 18-22 amino acids in length.

40. The non-human animal according to any one of claims 38 and 39, wherein the linker is a 2A peptide, and preferably F2A, E2A, P2A and T2A.

41. The non-human animal according to any one of claims 35 to 40, wherein the immunoglobulin heavy chain constant region gene is derived from the non-human animal.

42. The non-human animal according to any one of claims 35 to 41, comprising a human immunoglobulin heavy chain variable region gene operably linked downstream of the immunoglobulin heavy chain gene locus.

43. The non-human animal according to claim 42, comprising the gene(s) of one or multiple human heavy chain variable region V region(s), heavy chain variable region D region(s) or heavy chain variable region J region(s), or fragment(s) thereof operably linked downstream of the immunoglobulin heavy chain constant region gene locus.

44. The non-human animal according to claim 42, wherein the genes of the multiple human heavy chain variable region V regions, heavy chain variable region D regions, or heavy chain variable region J regions, or fragments thereof are directly linked.

45. The non-human animal according to any one of claims 34 to 44, comprising an endogenous immunoglobulin heavy chain variable region gene.

46. The non-human animal according to any one of claims 34 to 45, wherein the genomic integrity of the endogenous immunoglobulin heavy chain variable region gene sequence remains unchanged.

47. The non-human animal according to any one of claims 34 to 46, wherein the expression regulatory element for the endogenous immunoglobulin heavy chain variable region gene is functionally unimpaired.

48. The non-human animal according to any one of claims 34 to 47, comprising an expression regulatory element for complete endogenous immunoglobulin heavy chain variable region gene.

49. The non-human animal according to any one of claims 34 to 48, comprising complete endogenous immunoglobulin heavy chain variable region gene.

50. The non-human animal according to any one of claims 34 to 49, wherein the non-human animal does not express the endogenous immunoglobulin heavy chain variable region.

51. The non-human animal according to any one of claims 34 to 50, wherein the endogenous immunoglobulin variable region gene of the non-human animal does not express the antibody heavy chain variable region.

52. The non-human animal according to any one of claims 34 to 51, wherein the transcription direction of the human immunoglobulin heavy chain variable region gene is reverse to that of the endogenous immunoglobulin heavy chain variable region gene in the genome of the non-human animal.

53. The non-human animal according to any one of claims 34 to 52, wherein the immunoglobulin heavy chain constant region gene of the non-human animal is comprised between the human immunoglobulin heavy chain variable region gene and the endogenous immunoglobulin heavy chain variable region gene in the genome of the non-human animal.

54. The non-human animal according to any one of claims 34 to 53, wherein the transcription direction of the immunoglobulin heavy chain constant region gene is reverse to that of the endogenous immunoglobulin heavy chain variable region gene in the genome of the non-human animal.

55. The non-human animal according to any one of claims 34 to 54, wherein the transcription direction of the human immunoglobulin heavy chain variable region gene is the same as that of the immunoglobulin heavy chain constant region gene in the non-human animal.

56. The non-human animal according to any one of claims 34 to 55, wherein the chromosome of the non-human animal comprises sequentially, from upstream to downstream, the endogenous immunoglobulin heavy chain variable region gene, the immunoglobulin heavy chain constant region gene, and the human immunoglobulin heavy chain variable region gene.

57. The non-human animal according to any one of claims 34 to 56, which is a rodent.

58. The non-human animal according to any one of claims 34 to 57, which is a mouse.

59. The non-human animal according to claim 58, which is a mouse, and comprising the human immunoglobulin heavy chain variable region gene between the chromosome locations chr12: 113,149,523-113,223,857.

60. The non-human animal according to any one of claims 58 and 59, which is a mouse, and comprising the human immunoglobulin heavy chain variable region gene inserted at the chromosome location chr12: 113,190,256.

61. The non-human animal according to any one of claims 34 to 60, comprising a human immunoglobulin light chain variable region gene operably linked downstream of the immunoglobulin light chain gene locus in the genome of the non-human animal.

62. The non-human animal according to claim 61, comprising: the gene(s) of one or multiple human light chain variable region V region(s) or light chain variable region J region(s), or fragment(s) thereof operably linked downstream of the immunoglobulin light chain constant region gene locus in the genome of the non-human animal.

63. The non-human animal according to claim 62, wherein the gene(s) of the one or multiple human light chain variable region V region(s) or light chain variable region J region(s), or fragment(s) is/are directly linked.

64. The non-human animal according to any one of claims 34 to 63, comprising an endogenous immunoglobulin light chain variable region gene.

65. The non-human animal according to any one of claims 34 to 64, wherein the genomic integrity of the endogenous immunoglobulin light chain variable region gene sequence remains unchanged.

66. The non-human animal according to any one of claims 34 to 65, wherein the expression regulatory element for the endogenous immunoglobulin light chain variable region gene is functionally unimpaired.

67. The non-human animal according to any one of claims 34 to 66, comprising an expression regulatory element for complete endogenous immunoglobulin light chain variable region gene.

68. The non-human animal according to any one of claims 34 to 67, comprising complete endogenous immunoglobulin light chain variable region gene.

69. The non-human animal according to any one of claims 34 to 68, wherein the non-human animal does not express the endogenous immunoglobulin light chain variable region.

70. The non-human animal according to any one of claims 34 to 69, wherein the endogenous immunoglobulin variable region gene does not express the antibody light chain variable region.

71. The non-human animal according to any one of claims 34 to 70, wherein the transcription direction of the human immunoglobulin light chain variable region gene is reverse to that of the endogenous immunoglobulin light chain variable region gene in the genome of the non-human animal.

72. The non-human animal according to any one of claims 34 to 71, wherein the chromosome of the non-human animal comprises sequentially, from upstream to downstream, the endogenous immunoglobulin light chain variable region gene, the immunoglobulin light chain constant region gene, and the human immunoglobulin light chain variable region gene.

73. The non-human animal according to any one of claims 34 to 72, wherein the transcription direction of the immunoglobulin light chain constant region gene is reverse to that of the endogenous immunoglobulin light chain variable region gene in the genome of the non-human animal.

74. The non-human animal according to any one of claims 34 to 73, wherein the transcription direction of the human immunoglobulin light chain variable region gene is the same as that of the immunoglobulin light chain constant region gene in the genome of the non-human animal.

75. The non-human animal according to any one of claims 34 to 74, wherein the distance between the human immunoglobulin light chain variable region gene and the endogenous immunoglobulin light chain variable region gene is 4 Kbp-42 Kbp.

76. The non-human animal according to any one of claims 34 to 75, which is a mouse, and comprising the human immunoglobulin light chain variable region gene between the chromosome locations chr6: 70,703,738-70,742,704.

77. The non-human animal according to any one of claims 34 to 76, which is a mouse, and comprising the human immunoglobulin light chain variable region gene at the chromosome location chr6: 70,706,267.

78. A non-human animal cell, having a genome with an immunoglobulin heavy chain constant region gene comprising the following exons: CH2, CH3, CH1, H, M1 and M2, and a gene L encoding a linker, wherein the exon order is H, CH2, CH3, M1, M2, L, and CH1 in sequence.

79. A method for preparing an antibody that specifically binds to an antigen, the method comprising: immunizing the non-human animal according to any one of claims 35 to 77 with the antigen.

80. The method according to claim 78, wherein the antibody is a heavy chain-only antibody (HcAb).

81. A method for preparing a sample, the method comprising: exposing the non-human animal according to any one of claims 35 to 77 to an antigen, and collecting the sample from the non-human animal.

82. The method according to claim 81, wherein the sample comprises immune cells.

83. The method according to any one of claims 81 and 82, wherein the sample comprises B cells.

84. The method according to any one of claims 81 to 83, wherein the sample is derived from bone marrow, spleen tissues, lymph nodes, spleen cells or peripheral lymphocytes.

85. A genetically modified non-human animal genome having an immunoglobulin heavy chain constant region gene comprising the following exons: CH2, CH3 and CH1, wherein the exon order is CH2, CH3 and CH1 in sequence.

86. The non-human animal genome according to claim 85, wherein the CH2, CH3 and CH1 are derived from IgG and/or IgM.

87. The non-human animal genome according to claim 86, wherein the IgG comprises IgG3, IgG1, IgG2A, IgG2B and/or IgG2C.

88. The non-human animal genome according to any one of claims 85 to 87, wherein the immunoglobulin heavy chain constant region gene further comprises the following exons: H, M1 and M2, and a gene L encoding a linker, and the exon order is H, CH2, CH3, M1, M2, L and CH1 in sequence.

89. The non-human animal genome according to claim 88, wherein the linker is a self-cleaving peptide, and preferably a self-cleaving peptide having 18-22 amino acids in length.

90. The non-human animal genome according to any one of claims 88 and 89, wherein the linker is a 2A peptide, and preferably F2A, E2A, P2A and T2A.

91. The non-human animal genome according to any one of claims 85 to 90, wherein the immunoglobulin heavy chain constant region gene is derived from the non-human animal.

92. The non-human animal genome according to any one of claims 85 to 91, comprising a human immunoglobulin heavy chain variable region gene operably linked downstream of the immunoglobulin heavy chain gene locus.

93. The non-human animal genome according to claim 92, comprising the gene(s) of one or multiple human heavy chain variable region V region(s), heavy chain variable region D region(s) or heavy chain variable region J region(s), or fragment(s) thereof operably linked downstream of the immunoglobulin heavy chain constant region gene locus.

94. The non-human animal genome according to claim 92, wherein the genes of the multiple human heavy chain variable region V regions, heavy chain variable region D regions, or heavy chain variable region J regions, or fragments thereof are directly linked.

95. The non-human animal genome according to any one of claims 84 to 94, comprising an endogenous immunoglobulin heavy chain variable region gene.

96. The non-human animal genome according to any one of claims 84 to 95, wherein the genomic integrity of the endogenous immunoglobulin heavy chain variable region gene sequence remains unchanged.

97. The non-human animal genome according to any one of claims 84 to 96, wherein the expression regulatory element for the endogenous immunoglobulin heavy chain variable region gene is functionally unimpaired.

98. The non-human animal genome according to any one of claims 84 to 97, comprising an expression regulatory element for complete endogenous immunoglobulin heavy chain variable region gene.

99. The non-human animal genome according to any one of claims 84 to 98, comprising complete endogenous immunoglobulin heavy chain variable region gene.

100. The non-human animal genome according to any one of claims 84 to 99, wherein the non-human animal genome does not express the endogenous immunoglobulin heavy chain variable region.

101. The non-human animal genome according to any one of claims 84 to 90, wherein the endogenous immunoglobulin variable region gene does not express the antibody heavy chain variable region.

102. The non-human animal genome according to any one of claims 84 to 91, wherein the transcription direction of the human immunoglobulin heavy chain variable region gene is reverse to that of the endogenous immunoglobulin heavy chain variable region gene.

103. The non-human animal genome according to any one of claims 84 to 92, wherein the immunoglobulin heavy chain constant region gene of the non-human animal is comprised between the human immunoglobulin heavy chain variable region gene and the endogenous immunoglobulin heavy chain variable region gene.

104. The non-human animal genome according to any one of claims 84 to 93, wherein the transcription direction of the immunoglobulin heavy chain constant region gene is reverse to that of the endogenous immunoglobulin heavy chain variable region gene.

105. The non-human animal genome according to any one of claims 84 to 94, wherein the transcription direction of the human immunoglobulin heavy chain variable region gene is the same as that of the immunoglobulin heavy chain constant region gene in the non-human animal.

106. The non-human animal genome according to any one of claims 84 to 95, comprising sequentially, from upstream to downstream, the endogenous immunoglobulin heavy chain variable region gene, the immunoglobulin heavy chain constant region gene, and the human immunoglobulin heavy chain variable region gene.

107. The non-human animal genome according to any one of claims 84 to 96, wherein the non-human animal is a rodent.

108. The non-human animal genome according to any one of claims 84 to 97, wherein the non-human animal is a mouse.

109. The non-human animal genome according to claim 98, wherein the non-human animal is a mouse, and the human immunoglobulin heavy chain variable region gene is comprised between the chromosome locations chr12: 113,149,523-113,223,857.

110. The non-human animal genome according to any one of claims 98 and 99, wherein the non-human animal is a mouse, and the human immunoglobulin heavy chain variable region gene is inserted at the chromosome location chr12: 113,190,256.

111. The non-human animal genome according to any one of claims 84 to 100, comprising a human immunoglobulin light chain variable region gene operably linked downstream of the immunoglobulin light chain gene locus.

112. The non-human animal genome according to claim 111, comprising: the gene(s) of one or multiple human light chain variable region V region(s) or light chain variable region J region(s), or fragment(s) thereof operably linked downstream of the immunoglobulin light chain constant region gene locus.

113. The non-human animal genome according to claim 112, wherein the gene(s) of the one or multiple human light chain variable region V region(s) or light chain variable region J region(s), or fragment(s) is/are directly linked.

114. The non-human animal genome according to any one of claims 84 to 113, comprising an endogenous immunoglobulin light chain variable region gene.

115. The non-human animal genome according to any one of claims 84 to 114, wherein the genomic integrity of the endogenous immunoglobulin light chain variable region gene sequence remains unchanged.

116. The non-human animal genome according to any one of claims 84 to 115, wherein the expression regulatory element for the endogenous immunoglobulin light chain variable region gene is functionally unimpaired.

117. The non-human animal genome according to any one of claims 84 to 116, comprising an expression regulatory element for complete endogenous immunoglobulin light chain variable region gene.

118. The non-human animal genome according to any one of claims 84 to 117, comprising complete endogenous immunoglobulin light chain variable region gene.

119. The non-human animal genome according to any one of claims 84 to 118, wherein the non-human animal genome does not express the endogenous immunoglobulin light chain variable region.

120. The non-human animal genome according to any one of claims 84 to 119, wherein the endogenous immunoglobulin variable region gene does not express the antibody light chain variable region.

121. The non-human animal genome according to any one of claims 84 to 120, wherein the transcription direction of the human immunoglobulin light chain variable region gene is reverse to that of the endogenous immunoglobulin light chain variable region gene.

122. The non-human animal genome according to any one of claims 84 to 121, wherein the chromosome of the non-human animal genome comprises sequentially, from upstream to downstream, the endogenous immunoglobulin light chain variable region gene, the immunoglobulin light chain constant region gene, and the human immunoglobulin light chain variable region gene.

123. The non-human animal genome according to any one of claims 84 to 122, wherein the transcription direction of the immunoglobulin light chain constant region gene is reverse to that of the endogenous immunoglobulin light chain variable region gene.

124. The non-human animal genome according to any one of claims 84 to 123, wherein the transcription direction of the human immunoglobulin light chain variable region gene is the same as that of the immunoglobulin light chain constant region gene.

125. The non-human animal genome according to any one of claims 84 to 124, wherein the distance between the human immunoglobulin light chain variable region gene and the endogenous immunoglobulin light chain variable region gene is 4 Kbp-42 Kbp.

126. The non-human animal genome according to any one of claims 84 to 125, wherein the non-human animal is a mouse, and the human immunoglobulin light chain variable region gene is comprised between the chromosome locations chr6: 70,703,738-70,742,704.

127. The non-human animal genome according to any one of claims 84 to126, wherein the non-human animal is a mouse, and the human immunoglobulin light chain variable region gene is comprised at the chromosome locations chr6: 70,706,267.

128. An engineered chromosome, comprising the non-human animal genome according to any one of claims 84 to 127.

129. A cell, comprising the engineered chromosome according to claim 128.
